# EUROPEAN PATENT SPECIFICATION

(11) **EP 2 575 858 B1**
(45) Date of publication and mention of the grant of the patent: **20.12.2017**
(21) Application number: 11790534.9
(22) Date of filing: 06.06.2011
(51) Int. Cl.: A61K 38/16, A61K 38/17, A61P 3/10, A61P 3/00, A61P 17/00

(54) **METHODS FOR TREATMENT OF NEPHROTIC SYNDROME AND RELATED CONDITIONS**
VERFAHREN ZUR BEHANDLUNG DES NEPHROTISCHEN SYNDROMS UND VERWANDTER ERKRANKUNGEN
MÉTHODES DE TRAITEMENT DU SYNDROME NÉPHROTIQUE ET ÉTATS APPARENTÉS

(30) Priority: 05.06.2010 US 351866 P
(43) Date of publication of application: 10.04.2013
(73) Proprietor: Chugh, Sumant S., River Forest IL 60305 (US)
(72) Inventor: Chugh, Sumant S., River Forest IL 60305 (US)
(74) Representative: Richards, William John
(86) International application number: PCT/US2011/039255
(87) International publication number: WO 2011/153525

(56) References cited:
- WO-A2-2006/014678
- W. YIN ET AL: "Genetic Variation in ANGPTL4 Provides Insights into Protein Processing and Function", JOURNAL OF BIOLOGICAL CHEMISTRY, vol. 284, no. 19, 7 March 2009 (2009-03-07), pages 13213-13222, XP055089200, ISSN: 0021-9258, DOI: 10.1074/jbc.M900553200
- HUA SUN ET AL: "Enhanced Expression of ANGPTL2 in the Microvascular Lesions of Diabetic Glomerulopathy", NEPHRON EXPERIMENTAL NEPHROLOGY, vol. 105, no. 4, 1 January 2007 (2007-01-01), pages e117-e123, XP055089275, ISSN: 1660-2129, DOI: 10.1159/000100493
- E-C. LEE ET AL: "Identification of a New Functional Domain in Angiopoietin-like 3 (ANGPTL3) and Angiopoietin-like 4 (ANGPTL4) Involved in Binding and Inhibition of Lipoprotein Lipase (LPL)", JOURNAL OF BIOLOGICAL CHEMISTRY, vol. 284, no. 20, 15 May 2009 (2009-05-15) , pages 13735-13745, XP055035574, ISSN: 0021-9258, DOI: 10.1074/jbc.M807899200
- STEFANO ROMEO ET AL: "Population-based resequencing of ANGPTL4 uncovers variations that reduce triglycerides and increase HDL", NATURE GENETICS, vol. 39, no. 4, 1 April 2007 (2007-04-01), pages 513-516, XP055089195, ISSN: 1061-4036, DOI: 10.1038/ng1984

### SEQUENCE LISTING

<110> The UAB Research Foundation Chugh, Sumant S.
<120> METHODS FOR TREATMENT OF NEPHROTIC SYNDROME AND RELATED CONDITIONS
<130> U2010-0044WO
<150> 61/351,866
   <151> 2010-06-05
<160> 22
<170> PatentIn version 3.5
<210> 1
   <211> 406
   <212> PRT
   <213> Homo sapiens
<400> 1
   Met Ser Gly Ala Pro Thr Ala Gly Ala Ala Leu Met Leu Cys Ala Ala
   1               5                   10                  15
   Thr Ala Val Leu Leu Ser Ala Gln Gly Gly Pro Val Gln Ser Lys Ser
               20                  25                  30
   Pro Arg Phe Ala Ser Trp Asp Glu Met Asn Val Leu Ala His Gly Leu
           35                  40                  45
   Leu Gln Leu Gly Gln Gly Leu Arg Glu His Ala Glu Arg Thr Arg Ser
       50                  55                  60
   Gln Leu Ser Ala Leu Glu Arg Arg Leu Ser Ala Cys Gly Ser Ala Cys
   65                  70                  75                  80
   Gln Gly Thr Glu Gly Ser Thr Asp Leu Pro Leu Ala Pro Glu Ser Arg
                   85                  90                  95
   Val Asp Pro Glu Val Leu His Ser Leu Gln Thr Gln Leu Lys Ala Gln
               100                 105                 110
   Asn Ser Arg Ile Gln Gln Leu Phe His Lys Val Ala Gln Gln Gln Arg
           115                 120                 125
   His Leu Glu Lys Gln His Leu Arg Ile Gln His Leu Gln Ser Gln Phe
       130                 135                 140
   Gly Leu Leu Asp His Lys His Leu Asp His Glu Val Ala Lys Pro Ala
   145                 150                 155                 160
   Arg Arg Lys Arg Leu Pro Glu Met Ala Gln Pro Val Asp Pro Ala His
                   165                 170                 175
   Asn Val Ser Arg Leu His Arg Leu Pro Arg Asp Cys Gln Glu Leu Phe
               180                 185                 190
   Gln Val Gly Glu Arg Gln Ser Gly Leu Phe Glu Ile Gln Pro Gln Gly
           195                 200                 205
   Ser Pro Pro Phe Leu Val Asn Cys Lys Met Thr Ser Asp Gly Gly Trp
       210                 215                 220
   Thr Val Ile Gln Arg Arg His Asp Gly Ser Val Asp Phe Asn Arg Pro
   225                 230                 235                 240
   Trp Glu Ala Tyr Lys Ala Gly Phe Gly Asp Pro His Gly Glu Phe Trp
                   245                 250                 255
   Leu Gly Leu Glu Lys Val His Ser Ile Thr Gly Asp Arg Asn Ser Arg
               260                 265                 270
   Leu Ala Val Gln Leu Arg Asp Trp Asp Gly Asn Ala Glu Leu Leu Gln
           275                 280                 285
   Phe Ser Val His Leu Gly Gly Glu Asp Thr Ala Tyr Ser Leu Gln Leu
       290                 295                 300
   Thr Ala Pro Val Ala Gly Gln Leu Gly Ala Thr Thr Val Pro Pro Ser
   305                 310                 315                 320
   Gly Leu Ser Val Pro Phe Ser Thr Trp Asp Gln Asp His Asp Leu Arg
                   325                 330                 335
   Arg Asp Lys Asn Cys Ala Lys Ser Leu Ser Gly Gly Trp Trp Phe Gly
               340                 345                 350
   Thr Cys Ser His Ser Asn Leu Asn Gly Gln Tyr Phe Arg Ser Ile Pro
           355                 360                 365
   Gln Gln Arg Gln Lys Leu Lys Lys Gly Ile Phe Trp Lys Thr Trp Arg
       370                 375                 380
   Gly Arg Tyr Tyr Pro Leu Gln Ala Thr Thr Met Leu Ile Gln Pro Met
   385                 390                 395                 400
   Ala Ala Glu Ala Ala Ser
                   405
<210> 2
   <211> 1967
   <212> DNA
   <213> Homo sapiens
<400> 2
   ataaaaaccg tcctcgggcg cggcggggag aagccgagct gagcggatcc tcacacgact 60
   gtgatccgat tctttccagc ggcttctgca accaagcggg tcttaccccc ggtcctccgc 120
   gtctccagtc ctcgcacctg gaaccccaac gtccccgaga gtccccgaat ccccgctccc 180
   aggctaccta agaggatgag cggtgctccg acggccgggg cagccctgat gctctgcgcc 240
   gccaccgccg tgctactgag cgctcagggc ggacccgtgc agtccaagtc gccgcgcttt 300
   gcgtcctggg acgagatgaa tgtcctggcg cacggactcc tgcagctcgg ccaggggctg 360
   cgcgaacacg cggagcgcac ccgcagtcag ctgagcgcgc tggagcggcg cctgagcgcg 420
   tgcgggtccg cctgtcaggg aaccgagggg tccaccgacc tcccgttagc ccctgagagc 480
   cgggtggacc ctgaggtcct tcacagcctg cagacacaac tcaaggctca gaacagcagg 540
   atccagcaac tcttccacaa ggtggcccag cagcagcggc acctggagaa gcagcacctg 600
   cgaattcagc atctgcaaag ccagtttggc ctcctggacc acaagcacct agaccatgag 660
   gtggccaagc ctgcccgaag aaagaggctg cccgagatgg cccagccagt tgacccggct 720
   cacaatgtca gccgcctgca ccggctgccc agggattgcc aggagctgtt ccaggttggg 780
   gagaggcaga gtggactatt tgaaatccag cctcaggggt ctccgccatt tttggtgaac 840
   tgcaagatga cctcagatgg aggctggaca gtaattcaga ggcgccacga tggctcagtg 900
   gacttcaacc ggccctggga agcctacaag gcggggtttg gggatcccca cggcgagttc 960
   tggctgggtc tggagaaggt gcatagcatc acgggggacc gcaacagccg cctggccgtg 1020
   cagctgcggg actgggatgg caacgccgag ttgctgcagt tctccgtgca cctgggtggc 1080
   gaggacacgg cctatagcct gcagctcact gcacccgtgg ccggccagct gggcgccacc 1140
   accgtcccac ccagcggcct ctccgtaccc ttctccactt gggaccagga tcacgacctc 1200
   cgcagggaca agaactgcgc caagagcctc tctggaggct ggtggtttgg cacctgcagc 1260
   cattccaacc tcaacggcca gtacttccgc tccatcccac agcagcggca gaagcttaag 1320
   aagggaatct tctggaagac ctggcggggc cgctactacc cgctgcaggc caccaccatg 1380
   ttgatccagc ccatggcagc agaggcagcc tcctagcgtc ctggctgggc ctggtcccag 1440
   gcccacgaaa gacggtgact cttggctctg cccgaggatg tggccgttcc ctgcctgggc 1500
   aggggctcca aggaggggcc atctggaaac ttgtggacag agaagaagac cacgactgga 1560
   gaagccccct ttctgagtgc aggggggctg catgcgttgc ctcctgagat cgaggctgca 1620
   ggatatgctc agactctaga ggcgtggacc aaggggcatg gagcttcact ccttgctggc 1680
   cagggagttg gggactcaga gggaccactt ggggccagcc agactggcct caatggcgga 1740
   ctcagtcaca ttgactgacg gggaccaggg cttgtgtggg tcgagagcgc cctcatggtg 1800
   ctggtgctgt tgtgtgtagg tcccctgggg acacaagcag gcgccaatgg tatctgggcg 1860
   gagctcacag agttcttgga ataaaagcaa cctcagaaca cttaaaaaaa aaaaaaaaaa     1920
   aaaaaaaaaa aaaaaaaaaa aaaaaaaaaa aaaaaaaaaa aaaaaaa                   1967
<210> 3
   <211> 368
   <212> PRT
   <213> Homo sapiens
<400> 3
   Met Ser Gly Ala Pro Thr Ala Gly Ala Ala Leu Met Leu Cys Ala Ala
   1               5                   10                  15
   Thr Ala Val Leu Leu Ser Ala Gln Gly Gly Pro Val Gln Ser Lys Ser
               20                  25                  30
   Pro Arg Phe Ala Ser Trp Asp Glu Met Asn Val Leu Ala His Gly Leu
           35                  40                  45
   Leu Gln Leu Gly Gln Gly Leu Arg Glu His Ala Glu Arg Thr Arg Ser
       50                  55                  60
   Gln Leu Ser Ala Leu Glu Arg Arg Leu Ser Ala Cys Gly Ser Ala Cys
   65                  70                  75                  80
   Gln Gly Thr Glu Gly Ser Thr Asp Leu Pro Leu Ala Pro Glu Ser Arg
                   85                  90                  95
   Val Asp Pro Glu Val Leu His Ser Leu Gln Thr Gln Leu Lys Ala Gln
               100                 105                 110
   Asn Ser Arg Ile Gln Gln Leu Phe His Lys Val Ala Gln Gln Gln Arg
           115                 120                 125
   His Leu Glu Lys Gln His Leu Arg Ile Gln His Leu Gln Ser Gln Phe
       130                 135                 140
   Gly Leu Leu Asp His Lys His Leu Asp His Glu Val Ala Lys Pro Ala
   145                 150                 155                 160
   Arg Arg Lys Arg Leu Pro Glu Met Ala Gln Pro Val Asp Pro Ala His
                   165                 170                 175
   Asn Val Ser Arg Leu His His Gly Gly Trp Thr Val Ile Gln Arg Arg
               180                 185                 190
   His Asp Gly Ser Val Asp Phe Asn Arg Pro Trp Glu Ala Tyr Lys Ala
           195                 200                 205
   Gly Phe Gly Asp Pro His Gly Glu Phe Trp Leu Gly Leu Glu Lys Val
       210                 215                 220
   His Ser Ile Thr Gly Asp Arg Asn Ser Arg Leu Ala Val Gln Leu Arg
   225                 230                 235                 240
   Asp Trp Asp Gly Asn Ala Glu Leu Leu Gln Phe Ser Val His Leu Gly
                   245                 250                 255
   Gly Glu Asp Thr Ala Tyr Ser Leu Gln Leu Thr Ala Pro Val Ala Gly
               260                 265                 270
   Gln Leu Gly Ala Thr Thr Val Pro Pro Ser Gly Leu Ser Val Pro Phe
           275                 280                 285
   Ser Thr Trp Asp Gln Asp His Asp Leu Arg Arg Asp Lys Asn Cys Ala
       290                 295                 300
   Lys Ser Leu Ser Gly Gly Trp Trp Phe Gly Thr Cys Ser His Ser Asn
   305                 310                 315                 320
   Leu Asn Gly Gln Tyr Phe Arg Ser Ile Pro Gln Gln Arg Gln Lys Leu
                   325                 330                 335
   Lys Lys Gly Ile Phe Trp Lys Thr Trp Arg Gly Arg Tyr Tyr Pro Leu
               340                 345                 350
   Gln Ala Thr Thr Met Leu Ile Gln Pro Met Ala Ala Glu Ala Ala Ser
           355                 360                 365
<210> 4
   <211> 1853
   <212> DNA
   <213> Homo sapiens
<400> 4
   ataaaaaccg tcctcgggcg cggcggggag aagccgagct gagcggatcc tcacacgact      60
   gtgatccgat tctttccagc ggcttctgca accaagcggg tcttaccccc ggtcctccgc     120
   gtctccagtc ctcgcacctg gaaccccaac gtccccgaga gtccccgaat ccccgctccc     180
   aggctaccta agaggatgag cggtgctccg acggccgggg cagccctgat gctctgcgcc     240
   gccaccgccg tgctactgag cgctcagggc ggacccgtgc agtccaagtc gccgcgcttt     300
   gcgtcctggg acgagatgaa tgtcctggcg cacggactcc tgcagctcgg ccaggggctg     360
   cgcgaacacg cggagcgcac ccgcagtcag ctgagcgcgc tggagcggcg cctgagcgcg     420
   tgcgggtccg cctgtcaggg aaccgagggg tccaccgacc tcccgttagc ccctgagagc     480
   cgggtggacc ctgaggtcct tcacagcctg cagacacaac tcaaggctca gaacagcagg     540
   atccagcaac tcttccacaa ggtggcccag cagcagcggc acctggagaa gcagcacctg     600
   cgaattcagc atctgcaaag ccagtttggc ctcctggacc acaagcacct agaccatgag     660
   gtggccaagc ctgcccgaag aaagaggctg cccgagatgg cccagccagt tgacccggct     720
   cacaatgtca gccgcctgca ccatggaggc tggacagtaa ttcagaggcg ccacgatggc     780
   tcagtggact tcaaccggcc ctgggaagcc tacaaggcgg ggtttgggga tccccacggc     840
   gagttctggc tgggtctgga gaaggtgcat agcatcacgg gggaccgcaa cagccgcctg     900
   gccgtgcagc tgcgggactg ggatggcaac gccgagttgc tgcagttctc cgtgcacctg     960
   ggtggcgagg acacggccta tagcctgcag ctcactgcac ccgtggccgg ccagctgggc    1020
   gccaccaccg tcccacccag cggcctctcc gtacccttct ccacttggga ccaggatcac    1080
   gacctccgca gggacaagaa ctgcgccaag agcctctctg gaggctggtg gtttggcacc    1140
   tgcagccatt ccaacctcaa cggccagtac ttccgctcca tcccacagca gcggcagaag    1200
   cttaagaagg gaatcttctg gaagacctgg cggggccgct actacccgct gcaggccacc    1260
   accatgttga tccagcccat ggcagcagag gcagcctcct agcgtcctgg ctgggcctgg    1320
   tcccaggccc acgaaagacg gtgactcttg gctctgcccg aggatgtggc cgttccctgc    1380
   ctgggcaggg gctccaagga ggggccatct ggaaacttgt ggacagagaa gaagaccacg    1440
   actggagaag ccccctttct gagtgcaggg gggctgcatg cgttgcctcc tgagatcgag    1500
   gctgcaggat atgctcagac tctagaggcg tggaccaagg ggcatggagc ttcactcctt    1560
   gctggccagg gagttgggga ctcagaggga ccacttgggg ccagccagac tggcctcaat    1620
   ggcggactca gtcacattga ctgacgggga ccagggcttg tgtgggtcga gagcgccctc    1680
   atggtgctgg tgctgttgtg tgtaggtccc ctggggacac aagcaggcgc caatggtatc    1740
   tgggcggagc tcacagagtt cttggaataa aagcaacctc agaacactta aaaaaaaaaa    1800
   aaaaaaaaaa aaaaaaaaaa aaaaaaaaaa aaaaaaaaaa aaaaaaaaaa aaa           1853
<210> 5
   <211> 405
   <212> PRT
   <213> Rattus norvegicus
<400> 5
   Met Arg Cys Ala Pro Thr Ala Gly Ala Ala Leu Val Leu Cys Ala Ala
   1               5                   10                  15
   Thr Ala Gly Leu Leu Ser Ala Gln Gly Arg Pro Ala Gln Pro Glu Pro
               20                  25                  30
   Pro Arg Phe Ala Ser Trp Asp Glu Met Asn Leu Leu Ala His Gly Leu
           35                  40                  45
   Leu Gln Leu Gly His Gly Leu Arg Glu His Val Glu Arg Thr Arg Gly
       50                  55                  60
   Gln Leu Gly Ala Leu Glu Arg Arg Met Ala Ala Cys Gly Asn Ala Cys
   65                  70                  75                  80
   Gln Gly Pro Lys Gly Thr Asp Pro Lys Asp Arg Val Pro Glu Gly Gln
                   85                  90                  95
   Ala Pro Glu Thr Leu Gln Ser Leu Gln Thr Gln Leu Lys Ala Gln Asn
               100                 105                 110
   Ser Lys Ile Gln Gln Leu Phe Gln Lys Val Ala Gln Gln Gln Arg Tyr
           115                 120                 125
   Leu Ser Lys Gln Asn Leu Arg Ile Gln Asn Leu Gln Ser Gln Ile Asp
       130                 135                 140
   Leu Leu Thr Pro Thr His Leu Asp Asn Gly Val Asp Lys Thr Ser Arg
   145                 150                 155                 160
   Gly Lys Arg Leu Pro Lys Met Ala Gln Leu Ile Gly Leu Thr Pro Asn
                   165                 170                 175
   Ala Thr Arg Leu His Arg Pro Pro Arg Asp Cys Gln Glu Leu Phe Gln
               180                 185                 190
   Glu Gly Glu Arg His Ser Gly Leu Phe Gln Ile Gln Pro Leu Gly Ser
           195                 200                 205
   Pro Pro Phe Leu Val Asn Cys Glu Met Thr Ser Asp Gly Gly Trp Thr
       210                 215                 220
   Val Ile Gln Arg Arg Leu Asn Gly Ser Val Asp Phe Asn Gln Ser Trp
   225                 230                 235                 240
   Glu Ala Tyr Lys Asp Gly Phe Gly Asp Pro Gln Gly Glu Phe Trp Leu
                   245                 250                 255
   Gly Leu Glu Lys Met His Ser Ile Thr Gly Asp Arg Gly Ser Gln Leu
               260                 265                 270
   Ala Val Gln Leu Gln Asp Trp Asp Gly Asn Ala Lys Leu Leu Gln Phe
           275                 280                 285
   Pro Ile His Leu Gly Gly Glu Asp Thr Ala Tyr Ser Leu Gln Leu Thr
       290                 295                 300
   Glu Pro Thr Ala Asn Glu Leu Gly Ala Thr Asn Val Ser Pro Asn Gly
   305                 310                 315                 320
   Leu Ser Leu Pro Phe Ser Thr Trp Asp Gln Asp His Asp Leu Arg Gly
                   325                 330                 335
   Asp Leu Asn Cys Ala Lys Ser Leu Ser Gly Gly Trp Trp Phe Gly Thr
               340                 345                 350
   Cys Ser His Ser Asn Leu Asn Gly Gln Tyr Phe His Ser Ile Pro Arg
           355                 360                 365
   Gln Arg Gln Gln Arg Lys Lys Gly Ile Phe Trp Lys Thr Trp Lys Gly
       370                 375                 380
   Arg Tyr Tyr Pro Leu Gln Ala Thr Thr Leu Leu Ile Gln Pro Met Glu
   385                 390                 395                 400
   Ala Thr Ala Ala Ser
                   405
<210> 6
   <211> 1218
   <212> DNA
   <213> Rattus norvegicus
<400> 6
   atgcgctgcg ctccgaccgc aggcgctgct ctagtgctat gcgcagctac tgcggggctg 60
   ctgagcgcgc aagggcgccc tgcacagccg gagccgccgc gcttcgcatc ctgggatgaa 120
   atgaacttgc tggctcacgg gctgctgcag ctcggtcacg ggctgcggga acacgtggag 180
   cgcacccgtg gacagctggg cgcgctggaa cgccgcatgg ctgcctgcgg taacgcttgt 240
   caggggccca aggggacaga cccgaaggat agagtccccg aaggccaggc tcctgagact 300
   ctgcagagtt tacagactca actcaaggct cagaacagca agatccagca actgttccag 360
   aaggtagccc agcagcagag atacctatca aagcagaatc tgagaataca gaatcttcag 420
   agccagattg acctcttgac ccccacacac ctagacaatg gggtagacaa gacttcgagg 480
   ggaaagaggc ttcccaagat ggcccagctc attggcttga ctcccaacgc cacccgctta 540
   cacaggcctc cccgggactg ccaggaactc tttcaagaag gggagcggca cagtggactt 600
   ttccagatcc agcctctggg atctccacca tttttggtca actgtgagat gacttcagat 660
   ggaggctgga cggtgattca gagacgcctg aacggctctg tggacttcaa tcagtcttgg 720
   gaagcctaca aagatggctt cggagatccc caaggcgagt tctggctggg cctagagaag 780
   atgcacagca tcacagggga ccgaggaagc cagttggctg tgcagctcca ggactgggat 840
   ggcaatgcca aattgctcca atttcctatc catttggggg gtgaggacac agcctacagc 900
   ctgcagctca ccgagcccac ggccaatgag ctgggtgcca ccaatgtttc ccccaatggc 960
   ctttccctgc ccttctctac ctgggaccaa gaccacgacc tccgagggga ccttaactgt 1020
   gccaagagcc tctctggtgg ctggtggttt ggcacctgca gccattccaa tctaaatgga 1080
   caatacttcc actctattcc acggcaacgg cagcagcgta aaaaggggat cttctggaaa 1140
   acatggaagg gccgctacta tccactacag gctaccaccc tgttgatcca gcccatggag 1200
   gctacagcag cctcttag 1218
<210> 7
   <211> 410
   <212> PRT
   <213> Mus musculus
<400> 7
   Met Arg Cys Ala Pro Thr Ala Gly Ala Ala Leu Val Leu Cys Ala Ala
   1               5                   10                  15
   Thr Ala Gly Leu Leu Ser Ala Gln Gly Arg Pro Ala Gln Pro Glu Pro
               20                  25                  30
   Pro Arg Phe Ala Ser Trp Asp Glu Met Asn Leu Leu Ala His Gly Leu
           35                  40                  45
   Leu Gln Leu Gly His Gly Leu Arg Glu His Val Glu Arg Thr Arg Gly
       50                  55                  60
   Gln Leu Gly Ala Leu Glu Arg Arg Met Ala Ala Cys Gly Asn Ala Cys
   65                  70                  75                  80
   Gln Gly Pro Lys Gly Lys Asp Ala Pro Phe Lys Asp Ser Glu Asp Arg
                   85                  90                  95
   Val Pro Glu Gly Gln Thr Pro Glu Thr Leu Gln Ser Leu Gln Thr Gln
               100                 105                 110
   Leu Lys Ala Gln Asn Ser Lys Ile Gln Gln Leu Phe Gln Lys Val Ala
           115                 120                 125
   Gln Gln Gln Arg Tyr Leu Ser Lys Gln Asn Leu Arg Ile Gln Asn Leu
       130                 135                 140
   Gln Ser Gln Ile Asp Leu Leu Ala Pro Thr His Leu Asp Asn Gly Val
   145                 150                 155                 160
   Asp Lys Thr Ser Arg Gly Lys Arg Leu Pro Lys Met Thr Gln Leu Ile
                   165                 170                 175
   Gly Leu Thr Pro Asn Ala Thr His Leu His Arg Pro Pro Arg Asp Cys
               180                 185                 190
   Gln Glu Leu Phe Gln Glu Gly Glu Arg His Ser Gly Leu Phe Gln Ile
           195                 200                 205
   Gln Pro Leu Gly Ser Pro Pro Phe Leu Val Asn Cys Glu Met Thr Ser
       210                 215                 220
   Asp Gly Gly Trp Thr Val Ile Gln Arg Arg Leu Asn Gly Ser Val Asp
   225                 230                 235                 240
   Phe Asn Gln Ser Trp Glu Ala Tyr Lys Asp Gly Phe Gly Asp Pro Gln
                   245                 250                 255
   Gly Glu Phe Trp Leu Gly Leu Glu Lys Met His Ser Ile Thr Gly Asn
               260                 265                 270
   Arg Gly Ser Gln Leu Ala Val Gln Leu Gln Asp Trp Asp Gly Asn Ala
           275                 280                 285
   Lys Leu Leu Gln Phe Pro Ile His Leu Gly Gly Glu Asp Thr Ala Tyr
       290                 295                 300
   Ser Leu Gln Leu Thr Glu Pro Thr Ala Asn Glu Leu Gly Ala Thr Asn
   305                 310                 315                 320
   Val Ser Pro Asn Gly Leu Ser Leu Pro Phe Ser Thr Trp Asp Gln Asp
                   325                 330                 335
   His Asp Leu Arg Gly Asp Leu Asn Cys Ala Lys Ser Leu Ser Gly Gly
               340                 345                 350
   Trp Trp Phe Gly Thr Cys Ser His Ser Asn Leu Asn Gly Gln Tyr Phe
           355                 360                 365
   His Ser Ile Pro Arg Gln Arg Gln Glu Arg Lys Lys Gly Ile Phe Trp
       370                 375                 380
   Lys Thr Trp Lys Gly Arg Tyr Tyr Pro Leu Gln Ala Thr Thr Leu Leu
   385                 390                 395                 400
   Ile Gln Pro Met Glu Ala Thr Ala Ala Ser
                   405                 410
<210> 8
   <211> 1869
   <212> DNA
   <213> Mus musculus
<400> 8
   acgggctcca gatcttcttc tgcaccagag caagtctaag tctgagccgg ctcccccaga 60
   actccagctg ctgggtcttg aactcctgcg ttccggagtc ctagcgttgc tgcacccaag 120
   gccaccccca gaatcatgcg ctgcgctccg acagcaggcg ctgccctggt gctatgcgcg 180
   gctactgcgg ggcttttgag cgcgcaaggg cgccctgcac agccagagcc accgcgcttt 240
   gcatcctggg acgagatgaa cttgctggct cacgggctgc tacagctcgg ccatgggctg 300
   cgcgaacacg tggagcgcac ccgtgggcag ctgggcgcgc tggagcgccg catggctgcc 360
   tgtggtaacg cttgtcaggg gcccaaggga aaagatgcac ccttcaaaga ctccgaggat 420
   agagtccctg aaggccagac tcctgagact ctgcagagtt tgcagactca gctcaaggct 480
   caaaacagca agatccagca attgttccag aaggtggccc agcagcagag atacctatca 540
   aagcagaatc tgagaataca gaatcttcag agccagatag acctcttggc ccccacgcac 600
   ctagacaatg gagtagacaa gacttcgagg ggaaagaggc ttcccaagat gacccagctc 660
   attggcttga ctcccaacgc cacccactta cacaggccgc cccgggactg ccaggaactc 720
   ttccaagaag gggagaggca cagtggactt ttccagatcc agcctctggg gtctccacca 780
   tttttggtca actgtgagat gacttcagat ggaggctgga cagtgattca gagacgcctg 840
   aacggctctg tggacttcaa ccagtcctgg gaagcctaca aggatggctt cggagatccc 900
   caaggcgagt tctggctggg cctggaaaag atgcacagca tcacagggaa ccgaggaagc 960
   caattggctg tgcagctcca ggactgggat ggcaatgcca aattgctcca atttcccatc 1020
   catttggggg gtgaggacac agcctacagc ctgcagctca ctgagcccac ggccaatgag 1080
   ctgggtgcca ccaatgtttc ccccaatggc ctttccctgc ccttctctac ttgggaccaa 1140
   gaccatgacc tccgtgggga ccttaactgt gccaagagcc tctctggtgg ctggtggttt 1200
   ggtacctgta gccattccaa tctcaatgga caatacttcc actctatccc acggcaacgg 1260
   caggagcgta aaaagggtat cttctggaaa acatggaagg gccgctacta tcctctgcag 1320
   gctaccaccc tgctgatcca gcccatggag gctacagcag cctcttagcc tcctcactgg 1380
   agcctggttc caggcctaag aagacagtga ctttggttgt ggccctgaga tttggccatt 1440
   ctctgctggg ggcaggagct ctaagtaggg ctatctgcgt cttgtggaca aagaagaagc 1500
   ccgtaactgg agagactgga ggaccccttt tccgtgttgg ggtctgcaag cattgttgtc 1560
   tgaaacagtc agagcaacag gaaacaaatg gcccagatcc agaaaacatg ggctcgaggg 1620
   gcactgaata tcacttctcg cctaccagag aagttgggga tgcagaggga ccactacagt 1680
   ccaactagct gggcccttaa tggcggactc agtcatattg actgactgga gacagggtgc 1740
   caggagccct ggatacactc atggtgctgt tgtaggtgct gtggatgcac aggtgctaac 1800
   tgtggttccc aggcacaact cacagcattc ttacaataaa aacaacctca gaacaaaaaa 1860
   aaaaaaaaa 1869
<210> 9
   <211> 406
   <212> PRT
   <213> Homo sapiens
<220>
   <221> MISC_FEATURE
   <222> (39)..(39)
   <223> x = A, K, E or D
<220>
   <221> MISC_FEATURE
   <222> (40)..(40)
   <223> x = A, K, E or D
<220>
   <221> MISC_FEATURE
   <222> (76).. (76)
   <223> x = A or S
<220>
   <221> MISC_FEATURE
   <222> (80)..(80)
   <223> x = A or S
<220>
   <221> MISC_FEATURE
   <222> (161)..(161)
   <223> x = D, R, K, G, A, V or S
<220>
   <221> MISC_FEATURE
   <222> (162)..(162)
   <223> x = D, R, K, G, A, V or S
<220>
   <221> MISC_FEATURE
   <222> (163)..(163)
   <223> x = D, R, K, G, A, V or S
<220>
   <221> MISC_FEATURE
   <222> (164)..(164)
   <223> x = D, R, K, G, A, V or S
<220>
   <221> misc_feature
   <222> (221)..(221)
   <223> Xaa can be any naturally occurring amino acid
<400> 9
   Met Ser Gly Ala Pro Thr Ala Gly Ala Ala Leu Met Leu Cys Ala Ala
   1               5                   10                  15
   Thr Ala Val Leu Leu Ser Ala Gln Gly Gly Pro Val Gln Ser Lys Ser
               20                  25                  30
   Pro Arg Phe Ala Ser Trp Xaa Xaa Met Asn Val Leu Ala His Gly Leu
           35                  40                  45
   Leu Gln Leu Gly Gln Gly Leu Arg Glu His Ala Glu Arg Thr Arg Ser
       50                  55                  60
   Gln Leu Ser Ala Leu Glu Arg Arg Leu Ser Ala Xaa Gly Ser Ala Xaa
   65                  70                  75                  80
   Gln Gly Thr Glu Gly Ser Thr Asp Leu Pro Leu Ala Pro Glu Ser Arg
                   85                  90                  95
   Val Asp Pro Glu Val Leu His Ser Leu Gln Thr Gln Leu Lys Ala Gln
               100                 105                 110
   Asn Ser Arg Ile Gln Gln Leu Phe His Lys Val Ala Gln Gln Gln Arg
           115                 120                 125
   His Leu Glu Lys Gln His Leu Arg Ile Gln His Leu Gln Ser Gln Phe
       130                 135                 140
   Gly Leu Leu Asp His Lys His Leu Asp His Glu Val Ala Lys Pro Ala
   145                 150                 155                 160
   Xaa Xaa Xaa Xaa Leu Pro Glu Met Ala Gln Pro Val Asp Pro Ala His
                   165                 170                 175
   Asn Val Ser Arg Leu His Arg Leu Pro Arg Asp Cys Gln Glu Leu Phe
               180                 185                 190
   Gln Val Gly Glu Arg Gln Ser Gly Leu Phe Glu Ile Gln Pro Gln Gly
           195                 200                 205
   Ser Pro Pro Phe Leu Val Asn Cys Lys Met Thr Ser Xaa Gly Gly Trp
       210                 215                 220
   Thr Val Ile Gln Arg Arg His Asp Gly Ser Val Asp Phe Asn Arg Pro
   225                 230                 235                 240
   Trp Glu Ala Tyr Lys Ala Gly Phe Gly Asp Pro His Gly Glu Phe Trp
                   245                 250                 255
   Leu Gly Leu Glu Lys Val His Ser Ile Thr Gly Asp Arg Asn Ser Arg
               260                 265                 270
   Leu Ala Val Gln Leu Arg Asp Trp Asp Gly Asn Ala Glu Leu Leu Gln
           275                 280                 285
   Phe Ser Val His Leu Gly Gly Glu Asp Thr Ala Tyr Ser Leu Gln Leu
       290                 295                 300
   Thr Ala Pro Val Ala Gly Gln Leu Gly Ala Thr Thr Val Pro Pro Ser
   305                 310                 315                 320
   Gly Leu Ser Val Pro Phe Ser Thr Trp Asp Gln Asp His Asp Leu Arg
   325 330 335
   Arg Asp Lys Asn Cys Ala Lys Ser Leu Ser Gly Gly Trp Trp Phe Gly
   340 345 350
   Thr Cys Ser His Ser Asn Leu Asn Gly Gln Tyr Phe Arg Ser Ile Pro
   355 360 365
   Gln Gln Arg Gln Lys Leu Lys Lys Gly Ile Phe Trp Lys Thr Trp Arg
   370 375 380
   Gly Arg Tyr Tyr Pro Leu Gln Ala Thr Thr Met Leu Ile Gln Pro Met
   385 390 395 400
   Ala Ala Glu Ala Ala Ser
   405
<210> 10
   <211> 368
   <212> PRT
   <213> Homo sapiens
<220>
   <221> MISC_FEATURE
   <222> (39)..(39)
   <223> x = A, K, D or E
<220>
   <221> MISC_FEATURE
   <222> (40)..(40)
   <223> x = A, K, D or E
<220>
   <221> MISC_FEATURE
   <222> (76) .. (76)
   <223> x = A or C
<220>
   <221> MISC_FEATURE
   <222> (80)..(80)
   <223> x = A or C
<220>
   <221> MISC_FEATURE
   <222> (161)..(161)
   <223> x = D, R, K, G, A, V or S
<220>
   <221> MISC_FEATURE
   <222> (162)..(162)
   <223> x = D, R, K, G, A, V or S
<220>
   <221> MISC_FEATURE
   <222> (163)..(163)
   <223> x = D, R, K, G, A, V or S
<220>
   <221> MISC_FEATURE
   <222> (164)..(164)
   <223> x = D, R, K, G, A, V or S
<400> 10
   Met Ser Gly Ala Pro Thr Ala Gly Ala Ala Leu Met Leu Cys Ala Ala
   1               5                   10                  15
   Thr Ala Val Leu Leu Ser Ala Gln Gly Gly Pro Val Gln Ser Lys Ser
               20                  25                  30
   Pro Arg Phe Ala Ser Trp Xaa Xaa Met Asn Val Leu Ala His Gly Leu
           35                  40                  45
   Leu Gln Leu Gly Gln Gly Leu Arg Glu His Ala Glu Arg Thr Arg Ser
       50                  55                  60
   Gln Leu Ser Ala Leu Glu Arg Arg Leu Ser Ala Xaa Gly Ser Ala Xaa
   65                  70                  75                  80
   Gln Gly Thr Glu Gly Ser Thr Asp Leu Pro Leu Ala Pro Glu Ser Arg
                   85                  90                  95
   Val Asp Pro Glu Val Leu His Ser Leu Gln Thr Gln Leu Lys Ala Gln
               100                 105                 110
   Asn Ser Arg Ile Gln Gln Leu Phe His Lys Val Ala Gln Gln Gln Arg
           115                 120                 125
   His Leu Glu Lys Gln His Leu Arg Ile Gln His Leu Gln Ser Gln Phe
       130                 135                 140
   Gly Leu Leu Asp His Lys His Leu Asp His Glu Val Ala Lys Pro Ala
   145                 150                 155                 160
   Xaa Xaa Xaa Xaa Leu Pro Glu Met Ala Gln Pro Val Asp Pro Ala His
                   165                 170                 175
   Asn Val Ser Arg Leu His His Gly Gly Trp Thr Val Ile Gln Arg Arg
               180                 185                 190
   His Asp Gly Ser Val Asp Phe Asn Arg Pro Trp Glu Ala Tyr Lys Ala
           195                 200                 205
   Gly Phe Gly Asp Pro His Gly Glu Phe Trp Leu Gly Leu Glu Lys Val
       210                 215                 220
   His Ser Ile Thr Gly Asp Arg Asn Ser Arg Leu Ala Val Gln Leu Arg
   225                 230                 235                 240
   Asp Trp Asp Gly Asn Ala Glu Leu Leu Gln Phe Ser Val His Leu Gly
                   245                 250                 255
   Gly Glu Asp Thr Ala Tyr Ser Leu Gln Leu Thr Ala Pro Val Ala Gly
               260                 265                 270
   Gln Leu Gly Ala Thr Thr Val Pro Pro Ser Gly Leu Ser Val Pro Phe
           275                 280                 285
   Ser Thr Trp Asp Gln Asp His Asp Leu Arg Arg Asp Lys Asn Cys Ala
       290                 295                 300
   Lys Ser Leu Ser Gly Gly Trp Trp Phe Gly Thr Cys Ser His Ser Asn
   305                 310                 315                 320
   Leu Asn Gly Gln Tyr Phe Arg Ser Ile Pro Gln Gln Arg Gln Lys Leu
                   325                 330                 335
   Lys Lys Gly Ile Phe Trp Lys Thr Trp Arg Gly Arg Tyr Tyr Pro Leu
               340                 345                 350
   Gln Ala Thr Thr Met Leu Ile Gln Pro Met Ala Ala Glu Ala Ala Ser
           355                 360                 365
<210> 11
   <211> 22
   <212> DNA
   <213> Homo sapiens
<400> 11
   tctgggatct ccaccatttt tg 22
<210> 12
   <211> 18
   <212> DNA
   <213> Homo sapiens
<400> 12
   tcaccgtcca gcctccat 18
<210> 13
   <211> 18
   <212> DNA
   <213> Homo sapiens
<400> 13
   caactgtgag atgacttc 18
<210> 14
   <211> 17
   <212> DNA
   <213> Homo sapiens
<400> 14
   cgccacccgc ttacaca 17
<210> 15
   <211> 22
   <212> DNA
   <213> Homo sapiens
<400> 15
   cagaggctgg atctggaaaa gt 22
<210> 16
   <211> 15
   <212> DNA
   <213> Homo sapiens
<400> 16
   tgccaggaac tcttt 15
<210> 17
   <211> 23
   <212> DNA
   <213> Homo sapiens
<400> 17
   tacaggctac caccctgttg atc 23
<210> 18
   <211> 17
   <212> DNA
   <213> Homo sapiens
<400> 18
   aaccgcgggc cctctag 17
<210> 19
   <211> 17
   <212> DNA
   <213> Homo sapiens
<400> 19
   ccatggaggc tacagca 17
<210> 20
   <211> 27
   <212> DNA
   <213> Homo sapiens
<400> 20
   cttgaaggga ttgaaaagat aattagc 27
<210> 21
   <211> 24
   <212> DNA
   <213> Homo sapiens
<400> 21
   ccatgagtca gaaaagcatt gaac 24
<210> 22
   <211> 17
   <212> DNA
   <213> Homo sapiens
<400> 22
   aggtgagcat tttcctg 17

## Description

### FIELD OF THE DISCLOSURE

The present disclosure is directed to methods for the treatment and prevention of nephrotic syndrome and conditions related thereto, such as, but not limited to, proteinuria and edema.

### BACKGROUND

Nephrotic syndrome (NS) is a general term that refers to the loss of protein in the urine (proteinuria), hyperlipidemia (hypercholesterolemia and hypertriglyceridemia), and edema. Nephrotic syndrome involves changes in the pathology of cells in the kidney, such as podocytes. Proteinuria is defined as the presence of an excess of serum proteins in the urine. Albuminuria, a specific type of proteinuria, is a pathological condition wherein albumin is present in the urine.

Podocytes (or visceral epithelial cells) are cells in the outer layer of the glomerular capillary loop in the kidneys. The glomerulus filters blood, holding back large molecules such as proteins, and passing through small molecules such as water, salts, and sugar, as the first step in forming urine. The long processes, or "foot projections," of the podocytes wrap around the capillaries, and come to rest on the glomerular basement membrane. The foot processes are connected by a porous structure called the slit diaphragm. The innermost layer of the glomerular capillary loop is made of fenestrated endothelial cells. Kidneys affected by nephrotic syndrome have abnormalities in the glomerular capillary loop that cause leakage of blood proteins, resulting in proteinuria.

When protein is lost in the urine, its plasma concentration decreases, allowing water to move into other areas of the body, which leads to swelling known as edema. Edema is commonly observed in the feet and legs, in the belly or abdomen (ascites), and around the eyes, but can occur anywhere, especially in response to gravity. Additionally, because of this extra fluid that stays in the body, people often gain weight, experience fatigue and may find that they urinate less often

Many conditions are categorized as nephrotic syndromes, including minimal change disease (MCD), focal segmental glomerulosclerosis (FSGS), membranous nephropathy (MN) (also called membranous glomerulonephritis, MGN), and membranoproliferative glomerulonephritis (MPGN). For years pathologists found no changes in MCD tissue when viewing specimens under light microscopy, hence the name minimal change disease. With the advent of electron microscopy, the changes now known as the hallmarks for the disease include diffuse loss of podocyte foot processes, vacuolation of the podocyte foot processes, and growth of microvilli on the visceral epithelial cells. Diabetic nephropathy is the most common cause of nephrotic syndrome.

Hypertriglyceridemia may occur due to changes in the activity of enzymes that degrade triglycerides, such as lipoprotein lipase (LPL) (2-4). Certain proteins involved in the etiology of nephrotic syndrome and proteinuria, such angiopoietin-like 4 (Angpt14), inhibit the activity of LPL.

The molecular basis of nephrotic syndrome is not known. Increased levels of Angpt14 have been noted in nephrotic syndrome, such as MCD, MN/MGN, and MPGN, but increased circulating levels of Angpt14 have not been associated with causation of proteinuria in nephrotic syndrome. However, the role of Angpt14 in nephrotic syndrome, such as but not limited to, MCD, FSGS, MN/MGN, and MPGN, and related conditions, such as, but not limited to, proteinuria have not been previously reported. Furthermore, the association of proteinuria and glucocorticoid sensitivity in nephrotic syndrome and the link between proteinuria and hypertriglyceridemia, two key components of nephrotic syndrome, have yet to be established. Therapy designed to reduce proteinuria further complicates the study of disease mechanisms. For example, glucocorticoids used to treat proteinuria in MCD independently raise plasma triglyceride levels (5), and normalization of plasma triglyceride levels lags behind the response of proteinuria to glucocorticoids in certain forms of nephrotic syndrome, such as MCD (6).

The present disclosure show that increased circulating levels of Angpt14 reduce the severity of nephrotic syndrome and conditions associated therewith, such as but not limited to, proteinuria. As a result, the present disclosure provides method for treating and/or preventing nephrotic syndrome, such as but not limited to, MCD, FSGS, MN/MGN, MPGN and diabetic nephropathy as well as methods of alleviating symptoms associated with nephrotic syndrome, including, but not limited to, proteinuria and edema. The present disclosure further provides methods for reducing proteinuria and edema.

WO2006014678 discloses compositions and methods of using ANGPTL4 and agonists and antagonists thereof, for the diagnosis and treatment of diseases or disorders.
YIN et al (Journal Of Biological Chemistry 2009, vol. 284, no. 19, pages 13213 - 1322) disclose that genetic variation in ANGPTL4 provides insights into protein processing and function.
SUN et al (Nephron Experimental Nephrology 2007 vol. 105, no. 4, pages e117 - e123) disclose enhanced expression of ANGPTL2 in the microvascular lesions of diabetic glomerulopathy.
LEE et al (Journal Of Biological Chemistry 2009, vol. 284, no. 20, pages 13735 - 13745) disclose identification of a new functional domain in angiopoietin-like 3 (ANGPTL3) and angiopoietin-like 4 (ANGPTL4) involved in binding and inhibition of lipoprotein lipase (LPL).
ROMEO et al (Nature Genetics 2007, vol. 39, no. 4, pages 513 - 516) disclose that population-based resequencing of ANGPTL4 uncovers variations that reduce triglycerides and increase HDL.

### BRIEF DESCRIPTION OF THE FIGURES

FIG 1. shows the development and characterization of aP2-Angptl4 TG rats.
FIG.1A shows a 2D gel analysis of 200 *µ*g human plasma (n = 4 patients/group, cropped representative blots shown) and demonstrates the presence of increased circulating levels of Angptl4 in patients with minimal change disease (MCD) in relapse and in patients with membranous nephropathy (MN) (indicated by arrows), compared to patients with MCD in remission (i. e. non proteinuric patients).
FIG. 1B shows a transgenic (TG) rat model for adipose tissue specific over expression of Angptl4 (aP2-Angplt4 TG)..
FIG. 1C shows tissue specific over expression of Angptl4 mRNA (n = 3 rats/group) in aP2-Angptl4 TG rats. WAT is white adipose tissue, BAT is brown adipose tissue. *** P < 0.001.
FIG. 1D shows 2D gel electrophoresis of 200 *µ*g plasma, followed by Western blot for Angptl4 and demonstrates that heterozygous aP2-Angptl4 TG rats had higher circulating Angptl4 levels than wild type rats (age 3 months, n = 3 blots/group).
FIG. 1E shows 2D gel electrophoresis of 200 *µ*g plasma, followed by Western blot with the anti-V5 and anti-Angptl4 antibodies and demonstrates the presence of adipose tissue secreted V5-tagged Angptl4 in the plasma of aP2-Angptl4 TG rats.
FIG. IF shows 2D gel electrophoresis of anti-N-terminal Angptl4 immunoprecipitates from aP2-Angptl4 TG rat plasma followed by Western blotting using lectin SNA I and anti-Angptl4 antibodies and confirmed the presence of circulating sialylated Angptl4 in the aP2-Angptl4.
FIG. 1G shows PAS stained sections from 3 month old heterozygous aP2-Angptl4 TG rats (n = 3 rats/group) and demonstrates normal glomerular morphology (magnification 400x).
FIG. 1H shows immunogold EM with anti-V5 antibody to specifically detect transgenic protein in 3 month heterozygous aP2-Angplt4 TG male rats and demonstrated gold particles selectively on the endothelial surface in aP2-Angptl4 TG rats (indicated by arrows).
FIG. 2 shows the relationship of increased circulating levels of Angptl4 with proteinuria/ albuminuria.
FIG. 2A shows assessment of urinary protein excretion (3 *µ*g / lane, except MCD remission) in different human and experimental disease conditions by GelCode blue stained SDS PAGE and demonstrated the absence of significant proteinuria in aP2-Angptl4 TG rats (lane marked with *, arrow shows intact albumin at around 70 kDa).

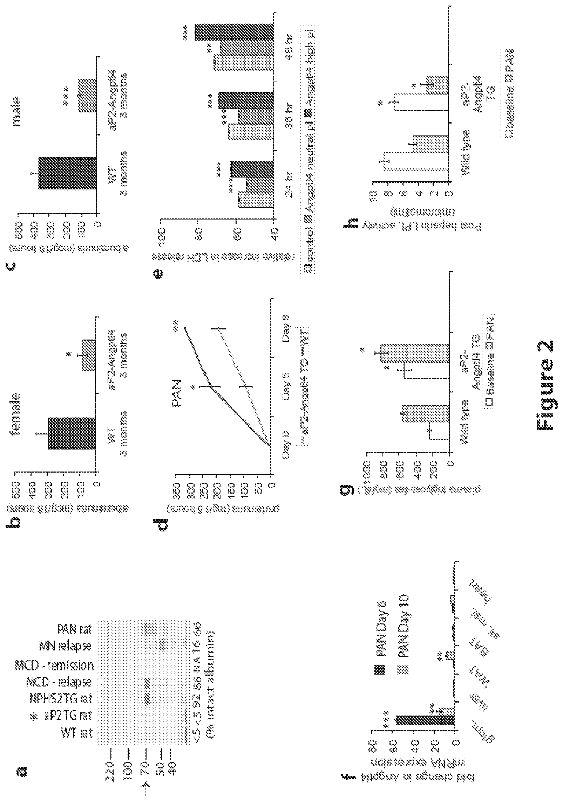

FIG. 2B shows assessment of albuminuria by ELISA and revealed that heterozygous female aP2-Angpt14 TG rats had lower albuminuria than wild type littermates (n = 6 rats/group). FIG. 2C shows assessment of albuminuria by ELISA and revealed that heterozygous male aP2-Angpt14 TG rats had lower albuminuria than wild type littermates (n = 6 rats/group). FIG. 2D shows induction of puromycin nephrosis (PAN), a model of nephrotic syndrome, in wild type and aP2-Angpt14 TG rats and demonstrates less proteinuria in aP2-Angpt14 TG rats compared to wild type littermates (n = 8 rats/group). * P < 0.05, ** P < 0.01 compared to corresponding controls
FIG. 2E shows recombinant Angpt14 had protective effects on cultured glomerular endothelial cells (GEnCs). ** P < 0.01, *** P < 0.001 compared to corresponding controls
FIG. 2F shows upregulation of Angpt14 in wild type rats in disease models like PAN on Day 6 was exclusively glomerular, while upregulation of Angpt14 in adipose tissue was noted on Day 10 when proteinuria and glomerular Angpt14 expression are on the decline (n = 3 rats / sample). ** P < 0.01 , *** P < 0.001 compared to corresponding controls
FIG. 2G shows increased circulating levels of Angpt14 at baseline and after induction of PAN in aP2-Angpt14 TG rats results in increased plasma triglyceride levels compared to wild type rats. * P < 0.05 compared to corresponding controls
FIG. 2H shows increased circulating levels of Angpt14 at baseline and after induction of PAN in aP2-Angpt14 TG rats results in reduced post-heparin lipoprotein lipase (LPL) activity compared to wild type rats. * P < 0.05 compared to corresponding controls
FIG. 3 show the primers and probes used for Taqman real time PCR (SEQ ID NOS. 11-22). FIG. 4 shows recombinant Angpt14 reduces proteinuria in animal models of human glomerular disease.
FIG. 4A shows reduction of proteinuria in Buffalo/Mna rats, a model of FSGS. In the Buffalo/Mna rat model, assessment of baseline proteinuria was made on Day 0. Angptl4 or control protein were injected intra-peritoneally on two consecutive days (Days 1 & 2, arrows) into Buffalo Mna rats (n=4 rats/group). Proteinuria was assessed on alternate days, and expressed as a percentage of baseline values. Significant reduction in proteinuria was noted in recombinant Angptl4 treated rats.
FIG. 4B shows reduction of proteinuria in Thy1.1 nephritis, a short term model of mesangial injury. Thy1.1 nephritis was induced in male Wistar rats (n=4 rats/group). After assessment of baseline proteinuria (Day 1), concentrated supernatant protein from Angpt14 stable or control cell lines were injected intra-venously on two consecutive days (Days 1 & 2, arrows) followed by assessment of proteinuria. Proteinuria was lower in Angpt14 treated rats throughout, and was statistically significant on Day 5. * P<0.05; ** P<0.01. all values are mean + SE

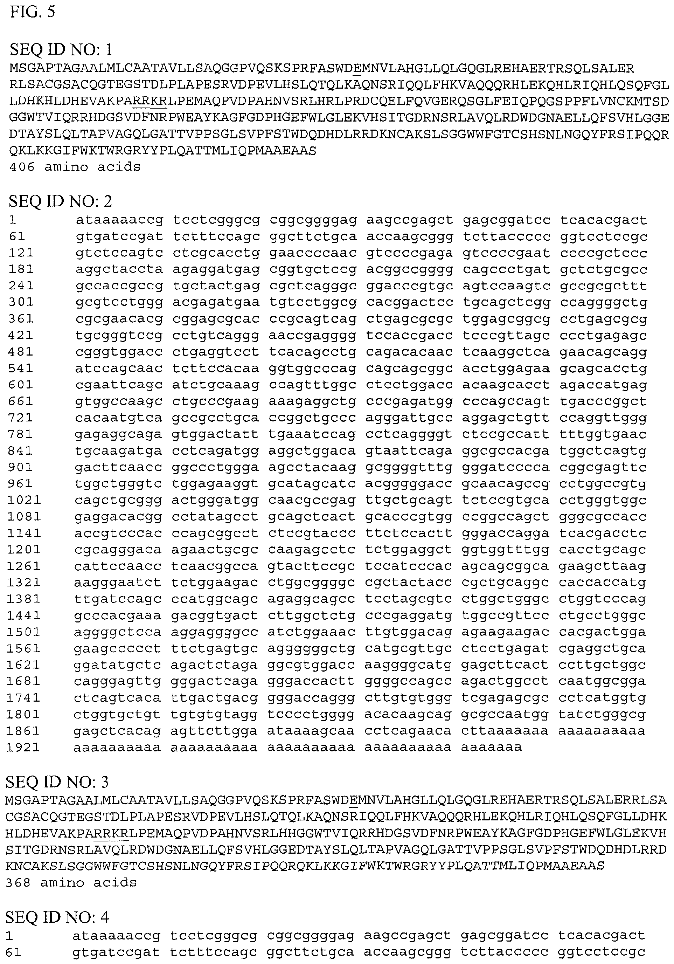

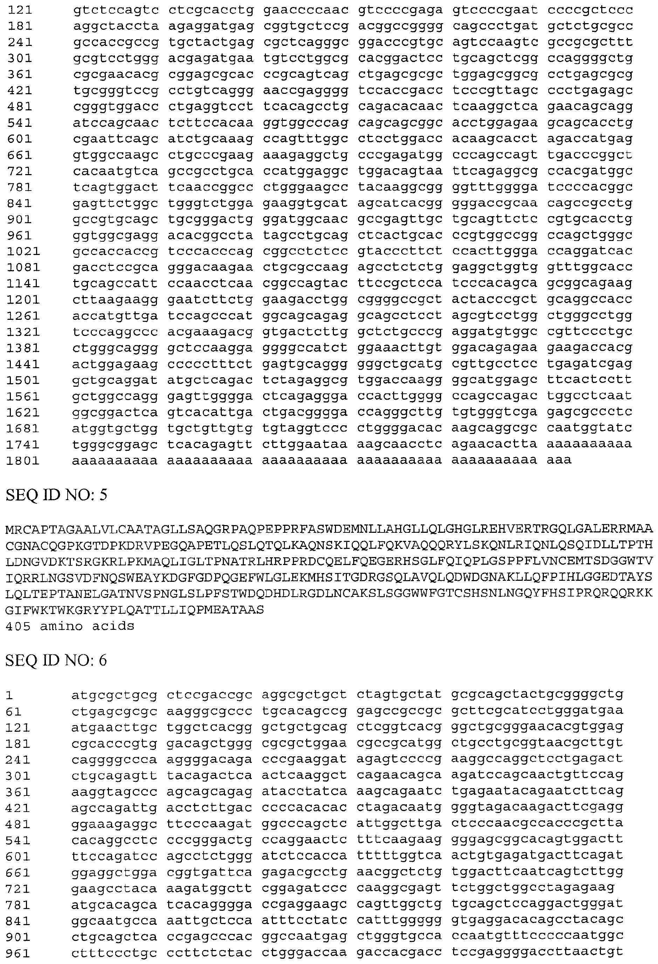

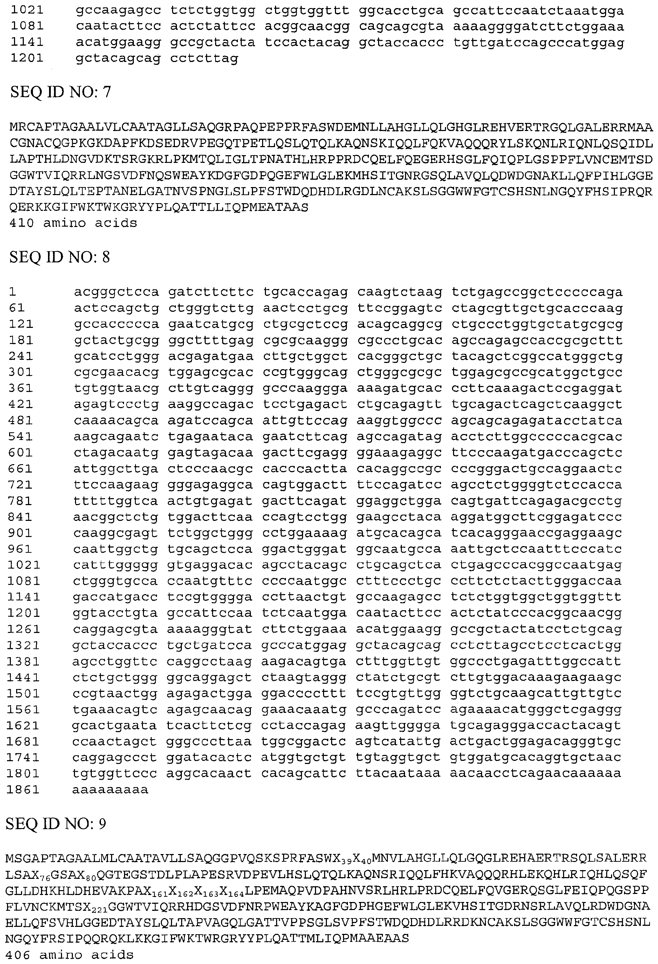

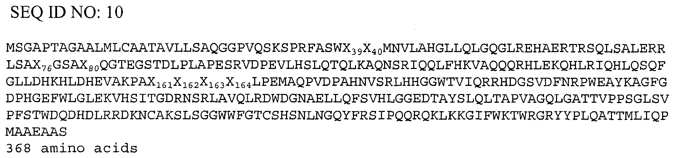

FIG. 5 shows the amino acid and cDNA sequences of Angpt14 from various species. SEQ ID NOS. 1 and 2 show amino acid and cDNA sequence from human (Protein Variant 1 isoform a, long form; underlined amino acid sequences at a position 40 and 161-164); SEQ ID NOS. 3 and 4 show amino acid and cDNA sequence from human (Protein Variant 3 isoform b, short form; underlined amino acid sequences at a position 40 and 161-164); SEQ ID NOS. 5 and 6 show amino acid and cDNA sequence from rat; SEQ ID NOS: 7 and 8 show amino acid and cDNA from mouse; underlined are forward sequencing primers. Bold are reverse sequencing primers.

### SUMMARY OF THE DISCLOSURE

In a first aspect, the present invention provides Angptl4 polypeptide for use in treatment or prevention of nephrotic syndrome.

In one embodiment, the nephrotic syndrome is characterized as MCD, FSGS, MN/MGN, MPGN or diabetic nephropathy. In another embodiment, the nephrotic syndrome is characterized as MCD. In a further embodiment, the nephrotic syndrome is characterized as MSGS. In a further embodiment, the nephrotic syndrome is caused by a diabetic condition. In one embodiment, the diabetic condition is diabetic nephropathy, diabetes mellitus, lupus nephritis or primary glomerular disease. Also described is the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type Angpt14 or an Angpt14 polypeptide derivative. In still a further embodiment, the Angpt14 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the Angplt4 polypeptides described herein. The Angpt14 polypeptide or polypeptide derivative may be administered at a therapeutically effective dose, either alone, as a part of a pharmaceutical composition or in combination with a secondary agent. In one embodiment, such administration treats nephrotic syndrome by providing Angpt14 function. In an alternate embodiment, such administration treats nephrotic syndrome by providing a modified Angpt14 function, such as, but not limited to, an Angpt14 function that display reduced LPL inhibition or is resistant to cleavage.

Also disclosed are methods of alleviating one or more symptoms of nephrotic syndrome, such as, but not limited to, proteinuria, hypercholesterolemia, hypertriglyceridemia and edema. In one embodiment, the nephrotic syndrome is characterized as MCD, FSGS, MN/MGN, MPGN and diabetic nephropathy. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. The methods comprise the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type
Angpt14. In still a further embodiment, the Angpt1 4 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the Angplt4 polypeptides described herein. The Angpt14 polypeptide or polypeptide derivative may be administered at a therapeutically effective dose, either alone, as a part of a pharmaceutical composition or in combination with a secondary agent. In one embodiment, such administration alleviates one or more symptoms of nephrotic syndrome by providing
Angpt14 function. In an alternate embodiment, such administration alleviates one or more symptoms of nephrotic syndrome by providing a modified Angpt14 function, such as, but not limited to, an Angpt14 function that display reduced LPL inhibition or is resistant to cleavage.

Also disclosed are methods for reducing proteinuria in a subject. In one embodiment, the subject is suffering from nephrotic syndrome. In one embodiment, the nephrotic syndrome is characterized as MCD, FSGS, MN/MGN, MPGN and diabetic nephropathy. In another embodiment, the nephrotic syndrome is characterized as MCD. In another embodiment, the subject is suffering from a disorder characterized by proteinuria. In another embodiment, the subject is suffering from a diabetic condition. In a further embodiment, the proteinuria is caused by FSGS. In one embodiment, the diabetic condition is diabetic nephropathy, diabetes mellitus, lupus nephritis or primary glomerular disease. The methods comprise the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type Angpt14. In still a further embodiment, the Angpt1 4 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the Angplt4 polypeptides described herein. The Angpt14 polypeptide or polypeptide derivative may be administered at a therapeutically effective dose, either alone, as a part of a pharmaceutical composition or in combination with a secondary agent. In one embodiment, such administration reduces proteinuria by providing Angpt14 function. In an alternate embodiment, such administration reduces proteinuria by providing a modified Angpt14 function, such as, but not limited to, an Angpt14 function that display reduced LPL inhibition or is resistant to cleavage.

Also disclosed are methods of reducing edema in a subject. In one embodiment, the subject is suffering from nephrotic syndrome. In one embodiment, the nephrotic syndrome is characterized as MCD, FSGS, MN/MGN, MPGN, and diabetic nephropathy. In another embodiment, the nephrotic syndrome is characterized as MCD. In a further embodiment, the nephrotic syndrome is caused by FSGS. In a specific embodiment, the edema is caused by decreased circulating levels of plasma proteins such as albumin. In a further embodiment, the nephrotic syndrome is caused by a diabetic condition In one embodiment, the diabetic condition is diabetic nephropathy, diabetes mellitus, lupus nephritis or primary glomerular disease. Reduction of proteinuria through the administration of an Angpt14 polypeptide of Angpt14 polypeptide derivative will reduce proteinuria, raise plasma protein levels and thereby reduce edema. The methods comprise the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type Angpt14. In still a further embodiment, the Angptl 4 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the AngplW polypeptides described herein. The Angpt14 polypeptide or polypeptide derivative may be administered at a therapeutically effective dose, either alone, as a part of a pharmaceutical composition or in combination with a secondary agent. In one embodiment, such administration reduces edema by providing Angpt14 function. In an alternate embodiment, such administration reduces edema by providing a modified Angpt14 function, such as, but not limited to, an Angpt14 function that display reduced LPL inhibition or is resistant to cleavage.

Also disclosed are methods of reducing hypercholesterolemia and/or hypertriglyceridemia in a subject. In one embodiment, the subject is suffering from nephrotic syndrome. In one embodiment, the nephrotic syndrome is characterized as MCD, FSGS, MN/MGN, MPGN and diabetic nephropathy. In another embodiment, the nephrotic syndrome is characterized as MCD. In a further embodiment, the nephrotic syndrome is caused by a diabetic condition In one embodiment, the diabetic condition is diabetic nephropathy, diabetes mellitus, lupus nephritis or primary glomerular disease. The methods comprise the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type Angpt14. In still a further embodiment, the Angpt1 4 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the Angplt4 polypeptides described herein. The Angpt14 polypeptide or polypeptide derivative may be administered at a therapeutically effective dose, either alone, as a part of a pharmaceutical composition or in combination with a secondary agent. In one embodiment, such administration reduces hypercholesterolemia and/or hypertriglyceridemia by providing
Angpt14 function. In an alternate embodiment, such administration reduces hypercholesterolemia and/or hypertriglyceridemia by providing a modified Angpt14 function, such as, but not limited to, an Angpt14 function that display reduced LPL inhibition or is resistant to cleavage.

Also disclosed are methods of treatment and/or prevention of a diabetic condition. In one embodiment, the diabetic condition is diabetic nephropathy, diabetes mellitus, lupus nephritis or primary glomerular disease. The methods comprise the step of administering to a subject an Angpt14 polypeptide or an Angpt14 polypeptide derivative. In one embodiment, the Angpt14 polypeptide comprises the sequence of SEQ ID NOS: 1, 3, 5, 7, 9 or 10. In an alternate, the amino acid sequence is a fragment of any of the foregoing sequences having an activity comparable to wild type Angpt14. In still a further embodiment, the Angpt1 4 polypeptide derivative is a derivative described herein and has been modified to have decreased LPL inhibitory activity, to be resistant to cleavage, or a combination of the foregoing. The Angpt14 polypeptide or polypeptide derivative, in one embodiment, is sialylated. Such derivative may be based on any of the Angplt4 polypeptides described herein. The Angpt14 polypeptide or polypeptide derivative may be administered at a therapeutically effective dose, either alone, as a part of a pharmaceutical composition or in combination with a secondary agent. In one embodiment, such administration treats the foregoing conditions by providing Angpt14 function. In an alternate embodiment, such administration treats the foregoing conditions by providing a modified Angpt14 function, such as, but not limited to, an Angpt14 function that display reduced LPL inhibition or is resistant to cleavage.

Also disclosed is a pharmaceutical composition for use in the methods as described above. The composition comprises one or more Anptl4 polypeptides or polypeptide derivatives. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein.

### DETAILED DESCRIPTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

While investigating nephrotic syndrome, it was noted that Angptl4 secreted from podocytes induced proteinuria. More importantly, as described herein, circulating Angptl4 reduced the proteinuria in a transgenic animal model. Increased levels of Angptl 4 have been noted in nephrotic syndrome, such as MCD and MN, but increased circulating levels of Angptl4 have not been associated with causation of nephrotic syndrome.

While increased Angptl4 levels are shown to treat nephrotic syndrome and reduce associated proteinuria, increased Angptl4 in the circulation has been observed to induce hyperlipidemia (hypertriglyceridemia), such as, but not limited to, through inhibition of LPL. It would be advantageous to provide the benefits of increased circulating Angptl4 levels without the negative consequences of hyperlipidemia. Such an approach is possible using the Angptl4 polypeptide derivatives as disclosed herein.

Angiopoietin-like proteins have been implicated in the development of hypertriglyceridemia and tumor metastasis, and are functionally distinct from the angiopoietins. Angptl4 is a PPARγ (8) and PPARα (9) target gene highly expressed in the liver and adipose tissue, strongly induced by fasting in white adipose tissue and liver, and is an apoptosis survival factor for vascular endothelial cells under normoxic conditions (10). Angptl4 is a potent inhibitor of LPL (11), inducing significant hypertriglyceridemia following intravenous injection or adenovirus-mediated expression (12, 13). Other studies showed lesser expression of Angptl4 in cardiomyocytes and skeletal muscle, and low level expression in whole kidney on Northern blot analysis (8). Recent population based studies of the *ANGPTL4* gene reveals variants that affect triglyceride levels in humans (14, 15).

The present disclosure shows a conclusive role for circulating Angptl4 in the reduction of proteinuria observed in nephrotic syndrome, such as, but not limited to, MCD, FSGS, MN, MPGN and diabetic nephropathy.

### Definitions

The terms "prevention", "prevent", "preventing", "suppression", "suppress" and "suppressing" as used herein refer to a course of action (such as administering a compound or pharmaceutical composition) initiated prior to the onset of a symptom, aspect, or characteristics of a disease or condition so as to prevent or reduce such symptom, aspect, or characteristics. Such preventing and suppressing need not be absolute to be useful.

The terms "treatment", "treat" and "treating" as used herein refers a course of action (such as administering a compound or pharmaceutical composition) initiated after the onset of a symptom, aspect, or characteristics of a disease or condition so as to eliminate or reduce such symptom, aspect, or characteristics. Such treating need not be absolute to be useful.

The term "in need of treatment" as used herein refers to a judgment made by a caregiver that a patient requires or will benefit from treatment. This judgment is made based on a variety of factors that are in the realm of a caregiver's expertise, but that includes the knowledge that the patient is ill, or will be ill, as the result of a disease or condition that is treatable by a method or compound of the disclosure.

The term "in need of prevention" as used herein refers to a judgment made by a caregiver that a patient requires or will benefit from prevention. This judgment is made based on a variety of factors that are in the realm of a caregiver's expertise, but that includes the knowledge that the patient will be ill or may become ill, as the result of a disease or condition that is preventable by a method or compound of the disclosure.

The term "individual", "subject" or "patient" as used herein refers to any animal, including mammals, such as mice, rats, other rodents, rabbits, dogs, cats, swine, cattle, sheep, horses, or primates, and humans. The term may specify male or female or both, or exclude male or female.

The term "therapeutically effective amount" as used herein refers to an amount of a compound, either alone or as a part of a pharmaceutical composition, that is capable of having any detectable, positive effect on any symptom, aspect, or characteristics of a disease or condition. Such effect need not be absolute to be beneficial. When referring to an Angptl4 polypeptide or Angptl4 polypeptide derivative, the term "therapeutically effective amount" refers to an amount of such polypeptide sufficient to reduce proteinuria in a subject.

The term "pharmaceutically acceptable derivative" means any pharmaceutically acceptable salt, ester, salt of an ester, solvate or other derivative of an Angptl4 polypeptide or polypeptide derivative of the present disclosure that, upon administration to a subject, is capable of providing (directly or indirectly) the function of wild type Angptl4; in certain embodiment, the Angptl4 polypeptide or polypeptide derivative shows decreased LPL inhibitory activity of a resistance to cleavage. Particularly favored derivatives are those that increase the bioavailability of an Angptl4 polypeptide or polypeptide derivative of the disclosure when such polypeptides are administered to a subject (e.g., by allowing an orally administered compound to be more readily absorbed into the blood), enhance delivery of such polypeptides to a given biological compartment, increase solubility to allow administration by injection, alter metabolism or alter rate of excretion. In one embodiment, the derivative is a prodrug.

The term "pharmaceutically acceptable salt(s)", unless otherwise indicated, includes salts of acidic or basic groups that may be present in the Angptl4 polypeptide or polypeptide derivative of the present disclosure.

The terms "about" and "approximately" shall generally mean an acceptable degree of error or variation for the quantity measured given the nature or precision of the measurements. Typical, exemplary degrees of error or variation are within 20 percent (%), preferably within 10%, and more preferably within 5% of a given value or range of values. For biological systems, the term "about" refers to an acceptable standard deviation of error, preferably not more than 2-fold of a give value. Numerical quantities given herein are approximate unless stated otherwise, meaning that the term "about" or "approximately" can be inferred when not expressly stated.

### Methods of Treatment and Prevention

The present disclosure provides methods of treatment and/or prevention of nephrotic syndrome. The present disclosure further provides methods of treatment and/or prevention of MCD, FSGS, and/or conditions with mesangial injury (such as diabetes mellitus). The present disclosure further provides methods of treatment and/or prevention of a diabetic condition. In one embodiment, the diabetic condition is diabetic nephropathy, diabetes mellitus, lupus nephritis or primary glomerular disease. The present disclosure additionally provides methods of alleviating one or more symptoms of nephritic syndrome, such as, but not limited to, proteinuria, hypercholesterolemia, hypertriglyceridemia and edema. Still further, the present disclosure provides for methods of reducing proteinuria. Further still, the present disclosure provides methods of reducing edema. The present disclosure additionally provides for pharmaceutical compositions comprising one or more Angptl4 polypeptides of Angptl4 polypeptide derivatives.

In one embodiment, the teachings of the present disclosure provide for the treatment and/or prevention of nephrotic syndrome in a subject in need of such treatment or prevention. In one embodiment, the nephrotic syndrome is characterized as MCD, FSGS, MN/MGN, and MPGN. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration treats nephrotic syndrome by providing Angptl4 function. In an alternate embodiment, such administration treats nephrotic syndrome by providing a modified Angptl4 function, such as, but not limited to, an Angptl4 function that display reduced LPL inhibition or is resistant to cleavage. Such method may further comprise identifying a subject in need of such treatment and/or prevention.

In an alternate embodiment, the teachings of the present disclosure provide for the treatment and/or prevention of MCD in a subject in need of such treatment or prevention. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration treats MCD by providing Angptl4 function. In an alternate embodiment, such administration treats MCD by providing a modified Angptl4 function, such as, but not limited to, an Angptl4 function that display reduced LPL inhibition or is resistant to cleavage. Such method may further comprise identifying a subject in need of such treatment and/or prevention.

In further embodiment, the teachings of the present disclosure provide for methods of alleviating one or more symptoms of nephrotic syndrome, such as, but not limited to, proteinuria, hypercholesterolemia, hypertriglyceridemia and edema. In one embodiment, the nephrotic syndrome is characterized as MCD, FSGS, MN/MGN, MPGN, and diabetic nephropathy. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration alleviates one or more symptoms of nephrotic syndrome by providing Angptl4 function. In an alternate embodiment, such administration alleviates one or more symptoms of nephrotic syndrome by providing a modified Angptl4 function, such as, but not limited to, an Angptl4 function that display reduced LPL inhibition or is resistant to cleavage. Such method may further comprise identifying a subject in need of such treatment and/or prevention.

In still a further embodiment, the teachings of the present disclosure provide methods for reducing proteinuria in a subject. In one embodiment, the subject is suffering from nephrotic syndrome. In one embodiment, the nephrotic syndrome is characterized as MCD, FSGS, MN/MGN, MPGN and diabetic nephropathy. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration reduces proteinuria by providing Angptl4 function. In an alternate embodiment, such administration reduces proteinuria by providing a modified Angptl4 function, such as, but not limited to, an Angptl4 function that display reduced LPL inhibition or is resistant to cleavage. Such method may further comprise identifying a subject in need of such treatment and/or prevention.

In yet a further embodiment, the teachings of the present disclosure provide methods for reducing edema in a subject. In one embodiment, the subject is suffering from nephrotic syndrome. In one embodiment, the nephrotic syndrome is characterized as MCD, FSGS, MN/MGN, MPGN and diabetic nephropathy. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. In a specific embodiment, the edema is caused by decreased circulating levels of plasma proteins such as albumin. Reduction of proteinuria through the administration of an Angptl4 polypeptide or a Angptl4 polypeptide derivative will raise reduce proteinuria, raise plasma protein levels and thereby reduce edema.The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration reduces edema by providing Angptl4 function. In an alternate embodiment, such administration reduces edema by providing a modified Angptl4 function, such as, but not limited to, an Angptl4 function that display reduced LPL inhibition or is resistant to cleavage. Such method may further comprise identifying a subject in need of such treatment and/or prevention.

In still a further embodiment, the teachings of the present disclosure provide methods for reducing hypercholesterolemia and/or hypertriglyceridemia in a subject. In one embodiment, the subject is suffering from nephrotic syndrome. In one embodiment, the nephrotic syndrome is characterized as MCD, FSGS, MN/MGN, and MPGN. In another embodiment, the nephrotic syndrome is characterized as MCD. In a further embodiment, the nephrotic syndrome is caused by FSGS. In a further embodiment, the nephrotic syndrome is caused by a diabetic condition. In one embodiment, the diabetic condition is diabetic nephropathy, diabetes mellitus, lupus nephritis or primary glomerular disease. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration reduces proteinuria by providing Angptl4 function. In an alternate embodiment, such administration reduces proteinuria by providing a modified Angptl4 function, such as, but not limited to, an Angptl4 function that display reduced LPL inhibition or is resistant to cleavage. Such method may further comprise identifying a subject in need of such treatment and/or prevention.

In still a further embodiment, the teachings of the present disclosure provide methods for treatment and/or prevention of a nephrotic syndrome that is caused by a diabetic condition. In one embodiment, the diabetic condition is diabetic nephropathy, diabetes mellitus, lupus nephritis or primary glomerular disease. The methods comprise the step of administering to a subject an Angptl4 polypeptide or an Angptl4 polypeptide derivative. In one embodiment, the Angptl4 polypeptide comprises the sequence of SEQ ID NOS: 1, 3, 5, 7, 9 or 10. In an alternate, the amino acid sequence is a fragment of any of the foregoing sequences having an activity comparable to wild type Angptl4. In still a further embodiment, the Angptl 4 polypeptide derivative is a derivative described herein and has been modified to have decreased LPL inhibitory activity, to be resistant to cleavage, or a combination of the foregoing. The Angptl4 polypeptide or polypeptide derivative, in one embodiment, is sialylated. Such derivative may be based on any of the Angplt4 polypeptides described herein. The Angptl4 polypeptide or polypeptide derivative may be administered at a therapeutically effective dose, either alone, as a part of a pharmaceutical composition or in combination with a secondary agent. In one embodiment, such administration treats the foregoing conditions by providing Angptl4 function. In an alternate embodiment, such administration treats the foregoing conditions by providing a modified Angptl4 function, such as, but not limited to, an Angptl4 function that display reduced LPL inhibition or is resistant to cleavage.

### Methods of Screening

The present disclosure also relates to a method for identifying a compound effective for treating or preventing nephrotic syndrome or a condition associated therewith, such as, but not limited to, proteinuria, hypercholesterolemia, hypertriglyceridemia or edema. In one embodiment, the nephrotic syndrome is characterized as MCD or MN. In another embodiment, the nephrotic syndrome is characterized as MCD. In another embodiment, the nephrotic syndrome is characterized by FSGS. In a further embodiment, the nephrotic syndrome is caused by a diabetic condition. In one embodiment, the diabetic condition is diabetic nephropathy, diabetes mellitus, lupus nephritis or primary glomerular disease. Such compounds may be useful as active ingredients included in pharmaceutical compositions or for administration alone. In one embodiment, the methods include determining the level a polypeptide involved in the etiology of nephrotic syndrome, such as, but not limited to, Angptl4.

In general, such screening methods comprises the steps of providing an assay system (as described in more detail below) that expresses a polypeptide involved in the etiology of nephrotic syndrome, such as, but not limited to, Angptl4, introducing into the assay system a test compound to be tested and determining whether the effect of the test compound on the level the polypeptide. The methods involve the identification of candidate or test compounds or agents (polypeptides, functional nucleic acids, carbohydrates, antibodies, small molecules or other molecules) which effect the the level of sialylation of the polypeptide. Such compounds may then be further tested in appropriate systems (such as, but not limited to, the animal models systems described herein) to determine the activity of the identified compounds.

Candidate compounds are identified using a variety of assays, such as, but not limited to, assays that employ cells which express a polypeptide involved in the etiology of nephrotic syndrome, such as, but not limited to, Angptl4 or in assays with isolated polypeptides. The various assays can employ a variety of variants of such polypeptides (e. g., full-length, a biologically active fragment, or a fusion protein which includes all or a portion of the desired polypeptide). Moreover, such polypeptides can be derived from any suitable mammalian species (e. g., human, rat or murine); in a specific embodiment, the polypeptide is derived from a human.

Where the assay involves the use of a whole cell, the cell may either naturally express a polypeptide involved in the etiology of nephrotic syndrome, such as, but not limited to, Angptl4, or may be modified to express the same. In the latter case, cells can be modified to express a desired polypeptide through conventional molecular biology techniques, such as by infecting the cell with a virus comprising such polypeptide. The cell can also be a prokaryotic or an eukaryotic cell that has been transfected with a nucleotide sequence encoding such polypeptide. In the foregoing, full length polypeptides, fragments or fusion proteins containing at least a part of such polypeptide may be used. Exemplary assay systems are described in the current specification.

The various screening assays may be combined with an *in vivo* assay entailing measuring the effect of the test compound on the symptoms the disease states and conditions discussed herein. In such an embodiment, the compounds may be evaluated to determine if they impact a parameter associated with nephrotic syndrome or a condition related thereto, such as, but not limited to, proteinuria or edema. Such parameters include, but are not limited to, determining 1) the level of a polypeptide involved in the etiology of nephrotic syndrome and related conditions, such as, but not limited to Angptl4 and 2) determining the level of protein excretion, either total or with regard to specific components.

In one embodiment, such a screening assay can be performed, for example, by determining the level of a polypeptide, such as, but not limited to, Angptl4 and detecting a difference in the level of such polypeptide in the presence of as compared to the absence of a test compound. Such screening assay may be in vitro, in vivo or ex vivo and may be cell culture based (either with whole cells or lysates) or may be based on an animal model. Any assay of the present disclosure may be used in the foregoing method.

Suitable test compounds for use in the screening methods can be obtained from any suitable source, such as conventional compound libraries. The test compounds can also be obtained using any of the numerous approaches in combinatorial library methods known in the art, including: biological libraries, spatially addressable parallel solid phase or solution phase libraries, synthetic library methods requiring deconvolution, the "one-bead one-compound" library method and synthetic library methods using affinity chromatography selection. The biological library approach is limited to peptide libraries, while the other four approaches are applicable to peptide, non-peptide oligomer or small molecule libraries of compounds. Examples of methods for the synthesis of molecular libraries can be found in the art. Libraries of compounds may be presented in solution or on beads, bacteria, spores, plasmids or phage.

The present disclosure also provides kits for carrying out any method of the present disclosure, which can contain any of the compounds and/or compositions disclosed herein or otherwise useful for practicing a method of the disclosure.

### Creation and Selection of Angptl4 polypeptide derivatives

Angiopoietin-related protein 4 is a polypeptide that in humans is encoded by the *ANGPTL4* gene. This gene is a member of the angiopoietin/angiopoietin-like gene family and encodes a glycosylated, secreted protein with a N-terminal signal sequence (amino acid residues 1-22 of SEQ ID NO:1), a coiled-coil domain (amino acid residues 23-170 of SEQ ID NO:1), a linker region (amino acid residues 171-185 of SEQ ID NO:1) and a fibrinogen C-terminal domain (amino acid residues 186-406 of SEQ ID NO:1). This gene is induced under hypoxic conditions in endothelial cells and is the target of peroxisome proliferation activators. The encoded protein is a serum hormone directly involved in regulating glucose homeostasis, lipid metabolism, and insulin sensitivity and also acts as an apoptosis survival factor for vascular endothelial cells. Alternatively spliced transcript variants encoding different isoforms have been described. This gene was previously referred to as ANGPTL2 but has been renamed ANGPTL4

Angptl4 inhibits LPL by breaking the LPL dimer molecule. Angptl4 has been unambiguously established as potent inhibitors of blood plasma triglyceride (TG) clearance, causing elevation of plasma TG levels. Recent evidence indicates that variations in the sequence of the Angptl4 polypeptide impact the effect on triglycerides, with certain mutations conferring reduced triglyceride levels implying a decreased inhibition of LPL (33 and 34, each of which are incorproated by reference for the teaching of Angptl4 variants). Furthermore, it has been reported that Angptl4 polypeptides exist in oligomeric forms and that oligomerization is required for inhibition of LPL activity. Once secreted from the cell, the oligomeric form is cleaved at a cleavage site (R₁₆₁RKR₁₆₄ of SEQ ID NOS: 1 and 3) to provide monomeric C-terminal forms and oligomeric N-terminal forms (34). The N-terminal residues 1-187 of the Angptl4 peptide were found to be sufficient to inhibit LPL (33).

The amino acid and cDNA sequences of the human, rat and mouse are provided in FIG. 5 and designated SEQ ID NOS: 1-8. The present disclosure contemplates the use of Angptl4 polypeptides and polypeptide derivatives in the methods disclosed herein, such as but not limited to, methods of treatment and prevention. As defined herein an Angptl4 polypeptide derivative refers to an Angptl4 polypeptide that includes one or more insertions, deletions and/or substitutions as determined from the amino acid sequence of the human polypeptides shown in SEQ ID NOS: 1 or 3 or the polypeptides shown in SEQ ID NOS: 5 or 7. In one embodiment, amino acid residues of the wild type Angptl 4 polypeptide are removed and replaced with different amino acid residues. The variants may be constructed as described herein or as known in the art. The variants so constructed may be evaluated using the methods and assays described herein to screen for activity.

When used herein, single letters when used to refer to amino acids have the following meanings:

| | | | |
|---|---|---|---|
| **G** | Glycine | **P** | Proline |
| **A** | Alanine | **V** | Valine |
| **L** | Leucine | **I** | Isoleucine |
| **M** | Methionine | **C** | Cysteine |
| **F** | Phenylalanine | **Y** | Tyrosine |
| **W** | Tryptophan | **H** | Histidine |
| **K** | Lysine | **R** | Arginine |
| **Q** | Glutamine | **N** | Asparagine |
| **E** | Glutamic Acid | **D** | Aspartic Acid |
| **S** | Serine | **T** | Threonine |

In one embodiment, the variant comprises a change in the amino acid sequence of an Angptl4 polypeptide that decreases the ability of Angptl4 to inhibit LPL or to or to be resistant to cleavage. The change may be a replacement, deletion and/or substitution of one or more residues in this region. Such changes have been described in the art (see references 33 and 34 which are herein incorporated by reference for such teaching). In one embodiment, such change occurs in residues 1-187 with respect to SEQ ID NO: 1 or residues 1-182 of SEQ ID NO: 3. In an alternate embodiment, such change occurs at position 40 with respect to SEQ ID NOS: 1, 3, 5 or 7 or SEQ ID NOS: 1 or 3. In one embodiment, the amino acid at position 40 (a negatively charged glutamic acid residue in wild-type Angptl4) is replaced with a neutral amino acid or a positively charged amino acid. In a particular embodiment, the change is an E40K substitution. In another particular embodiment, the change is an E40A substitution. The E40K and E40A substitutions have been shown to reduce LPL inhibition by Angptl4, but not interfere with expression, secretion, processing and other functions of the polypeptide. In a further particular embodiment, the change at position 40 of SEQ ID NOS: 1 and 3 is selected from those shown in Table 1 below. In yet a further embodiment, the amino acid at position 39 (a negatively charged aspartic acid residue in wild-type Angptl4) is replaced with a neutral of positively charged amino acid. In one embodiment, the substitution is a D39K substitution of a D39A substitution. In a further particular embodiment, the change at position 39 of SEQ ID NOS: 1 and 3 is selected from those shown in Table 1 below. In certain embodiments, a polypeptide variant may contain one of the aforementioned changes at position 40, one of the aforementioned changes at position 39 or a combination of the foregoing. In a particular embodiment, the polypeptide contains a D39K substitution and a E40K substitution, a D39A substitution and a E40K substitution or a D39K substitution and an E40A substitution.

**Table 1**

| **Modifications of E₄₀ in the human Angptl4 protein** | | | | | | | | |
|---|---|---|---|---|---|---|---|---|
| G | P | V | L | I | M | C | F | Y |
| w | H | R | Q | N | S | T | | |

In another embodiment, the variant comprises one or more changes in a region of the Angptl4 polypeptide responsible for cleavage of the polypeptide. In one embodiment, this region is the R₁₆₁RKR₁₆₄ region of Angptl4. The change may be a replacement, deletion and/or substitution of one or more residues in this region. The R₁₆₁RKR₁₆₄ region has been shown to be responsible for cleavage of the oligomeric forms of Angptl4, releasing oligomers of the N-terminal sequences and monomers of the C-terminal sequence. Forms of Angptl4 with a mutated cleavage site were shown to accumulate at higher levels in the circulation than wild-type polypeptide. Furthermore, preventing cleavage of the Angptl4 polypeptide stabilizes the oligomeric forms of Angptl4 observed to be efficacious in the present disclosure. In one embodiment, all 4 amino acid residues of the R₁₆₁RKR₁₆₄ region are changed; in an alternate embodiment, 1, 2 or 3 amino acid residues of the R₁₆₁RKR₁₆₄ region are changed. In a further embodiment, the arginine residues at positions 161, 162 or 164 are independently substituted with glycine, alanine, valine or serine and the lysine residue at position 163 is substituted with glycine, alanine, valine or serine. In a specific embodiment the R₁₆₁RKR₁₆₄ amino acid sequence of SEQ ID NOS: 1 or 3 is replaced with G₁₆₁AAG₁₆₄; in a further specific embodiment, the R₁₆₁RKR₁₆₄ amino acid sequence of SEQ ID NOS: 1 or 3 is replaced with G₁₆₁SGS₁₆₄ Exemplary amino acid sequences for replacement of the entire R₁₆₁RKR₁₆₄ region of SEQ ID NOS: 1 or 3 is provided in Table 2 below.

**Table 2**

| **Modifications of 161RRKR164 in the human Angptl4 protein** | | | | | | | | |
|---|---|---|---|---|---|---|---|---|
| GAAG | GAAV | GAVV | GVVV | GAAA | AAVV | VVVV | GSGS | GSGG |
| GAGA | GAVA | GVAV | VGVV | AGAA | AAVA | SSSS | GSSG | SGGG |
| GGAA | GVAA | GVVA | VVVG | AAAG | AAAV | GGGG | GGSS | GGSG |
| AGGA | AGVA | AGVV | VVGV | AAGA | AVAA | AAAA | SGSG | GGGS |
| AGAG | AGAV | AVVG | | | VAAA | | SGGS | |
| AAGG | AAVG | AVGV | | | AVVV | | SSGG | |
| | AAGV | VGAV | | | VAVV | | | |
| | AVAG | VGVA | | | VVVA | | | |
| | AVGA | VAGV | | | VVAV | | | |
| | VGAA | VAVG | | | | | | |
| | VAAG | VVGA | | | | | | |
| | VAGA | VVAG | | | | | | |

In a further embodiment, one or more of the amino acids in the R₁₆₁RKR₁₆₄ amino acid sequence of SEQ ID NOS: 1 or 3 altered to remove a consensus binding site of an enzyme capable of cleaving Angplt4, such that Angptl4 is resistant to cleavage. In one embodiment, the enzyme is a proprotein convertase and the consensus binding site is RXKR, RXRR, RR or KR, where X is any amino acid. In making such alternations, one or more amino acids may be deleted or substituted with glycine, alanine, valine or serine or with any of the other substitutions discussed herein.

In still a further embodiment, the variant comprises one or more changes in a region of the Angptl4 polypeptide responsible for oligomerization of the polypeptide. In one embodiment, this region is the C₇₆ and/or C₈₀ region of Angptl4. The C₇₆ and/or C₈₀ region has been shown to be involved in oligomerization of the Angptl4 polypeptide (34, which reference is incorproated herein for such teaching). The change may be a replacement, deletion and/or substitution of one or more residues in this region. In a particular embodiment, only one of the cysteine residues at positions 76 and 80 is substituted; in an alternate embodiment, both cysteine residues at positions 76 and 80 are both substituted. In one embodiment, at least one of the cysteine residues at position 76 and 80 are substituted independently with alanine or serine; in another embodiment, both cysteine residues are substituted with alanine or serine.

In a further embodiment, the variant comprises one or more changes in the R₁₆₁RKR₁₆₄ region of Angplt4 that inhibits the cleavage of the Angpt14 polypeptide oligomer and a change at position 40 that reduces inhibition of LPL activity by Angpt14. Any of the changes discussed herein are included.

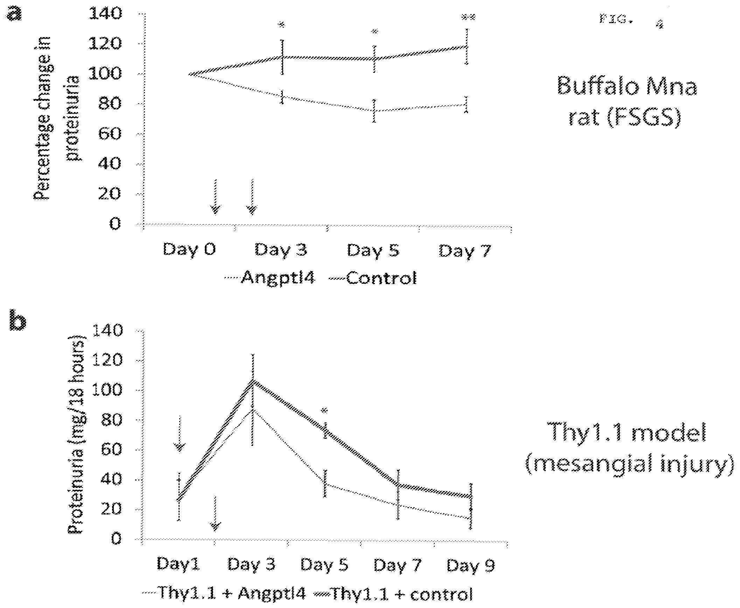

In one embodiment, the present disclosure provides for Angpt14 polypeptide variants having the amino acid sequence of SEQ ID NOS: 9 or 10. SEQ ID NO: 9 is shown in FIG.5 and includes the wild type sequence of Angpt14 from SEQ ID NO: 1, with the exception of substitutions at positions 39, 40, 76, 80 and 161-164 indicated by X39, X40, X76, X80, X161, X162, X163 and X164, respectively. SEQ ID NO: 10 is shown in FIG.4 and includes the wild type sequence of Angpt14 from SEQ ID NO: 3, with the exception of substitutions at positions 39, 40, 76, 80 and 161 -164 indicated by X39, X40, X76, X80, X161, X162, X163 and X164, respectively.

In SEQ ID NOS: 9 and 10, X39 may be A, G, P, V, L, I, M, C, F, Y, W, H, R, Q, N, S, T or K. In one embodiment, X39 is a neutral or positively charged amino acid. In a further embodiment, X39 may be A or K. In still a further embodiment, X39 may be D.

In SEQ ID NOS: 9 and 10, X40 may be A, G, P, V, L, I, M, C, F, Y, W, H, R, Q, N, S, T or K. In one embodiment, X40 is a neutral or positively charged amino acid. In a further embodiment, X40 may be A or K. In still a further embodiment, X40 may be E. In yet a further embodiment, X40 may be E when X39 is not D and X39 may be D when X40 is not E.

In SEQ ID NOS: 9 and 10, at least one of X76 and X80 may be substituted. In one embodiment, X76 and X80 are independently A or S or C. In one embodiment, one of X76 and X80 may be A or S and the other of X76 and X80 is C. In a further embodiment, both of X76 and X80 may be independently A or S. In still a further embodiment, both of X76 and X80 may C.

In SEQ ID NOS: 9 and 10, at least one of X161, X162, X163 and X164 may be substituted. In one embodiment, all 4 of X1 6 1, X162, X 1 03 and X164 are substituted; in an alternate embodiment. 1, 2 or 3 of X161, X162, Xi63 and X164 are substituted. In a further embodiment, X161, X162, X163 and X164 are independently D, R, K, G, A, V or S. In still a further embodiment, all 4 of are substituted with the combinations recited in Table 2.

The present disclosure contemplates combinations of the foregoing in any form. Furthermore, the designated residues in SEQ ID NOS: 9 and 10 may be substituted with conservative amino acid substitutions as designated in Table 3, or with residues having a difference in hydropathic index of +/- 1 or less or with residues having a difference in hydrophilicity values of +/- 1 or less.

In a one embodiment, X₃₉ is D, X₄₀ is A or K, X₇₆ and X₈₀ are C and X₁₆₁, X₁₆₂, X₁₆₃ and X₁₆₄ are independently substituted with D, R, K, G, A, V or S, optionally provided that at least one of X₁₆₁, X₁₆₂, X₁₆₃ and X₁₆₄ is an amino acid not found in SEQ ID NOS: 1 or 3. In another embodiment, X₃₉ is D, X₄₀ is A or K, X₇₆ and X₈₀ are C and X₁₆₁, X₁₆₂, X₁₆₃ and X₁₆₄ are selected from the combinations shown in Table 2. In still another embodiment, X₃₉ is D, X₄₀ is A or K, X₇₆ and X₈₀ are C and X₁₆₁, X₁₆₂, X₁₆₃ and X₁₆₄ are GSGS or GAAG.

In an additional embodiment, X₃₉ is D, X₄₀ is A or K, one of X₇₆ and X₈₀ is A or S and the other of X₇₆ and X₈₀ is C and X₁₆₁, X₁₆₂, X₁₆₃ and X₁₆₄ are independently substituted with D, R, K, G, A, V or S, optionally provided that at least one of X₁₆₁, X₁₆₂, X₁₆₃ and X₁₆₄ is an amino acid not found in SEQ ID NOS: 1 or 3. In a further embodiment, X₃₉ is D, X₄₀ is A or K, one of X₇₆ and X₈₀ is A or S and the other of X₇₆ and X₈₀ is C and X₁₆₁, X₁₆₂, X₁₆₃ and X₁₆₄ are selected from the combinations shown in Table 2. In still a further embodiment, X₃₉ is D, X₄₀ is A or K, one of X₇₆ and X₈₀ is A or S and the other of X₇₆ and X₈₀ is C and X₁₆₁, X₁₆₂, X₁₆₃ and X₁₆₄ are GSGS or GAAG.

In one embodiment, X₃₉ is A or K, X₄₀ is E, X₇₆ and X₈₀ are C and X₁₆₁, X₁₆₂, X₁₆₃ and X₁₆₄ are independently substituted with D, R, K, G, A, V or S, optionally provided that at least one of X₁₆₁, X₁₆₂, X₁₆₃ and X₁₆₄ is an amino acid not found in SEQ ID NOS: 1 or 3. In another embodiment, X₃₉ is A or K, X₄₀ is E, X₇₆ and X₈₀ are C and X₁₆₁, X₁₆₂, X₁₆₃ and X₁₆₄ are selected from the combinations shown in Table 2. In still another embodiment, X₃₉ is A or K, X₄₀ is E, X₇₆ and X₈₀ are C and X₁₆₁, X₁₆₂, X₁₆₃ and X₁₆₄ are GSGS or GAAG.

In one embodiment, X₃₉ is D, X₄₀ is K, X₇₆ and X₈₀ are C and X₁₆₁, X₁₆₂, X₁₆₃ and X₁₆₄ are independently substituted with D, R, K, G, A, V or S, optionally provided that at least one of X₁₆₁, X₁₆₂, X₁₆₃ and X₁₆₄ is an amino acid not found in SEQ ID NOS: 1 or 3. In another embodiment, X₃₉ is D, X₄₀ is K, X₇₆ and X₈₀ are C and X₁₆₁, X₁₆₂, X₁₆₃ and X₁₆₄ are selected from the combinations shown in Table 2. In still another embodiment, X₃₉ is D, X₄₀ is K, X₇₆ and X₈₀ are C and X₁₆₁, X₁₆₂, X₁₆₃ and X₁₆₄ are GSGS or GAAG.

In one embodiment, X₃₉ is D, X₄₀ is K, one of X₇₆ and X₈₀ is A or S and the other of X₇₆ and X₈₀ is C and X₁₆₁, X₁₆₂, X₁₆₃ and X₁₆₄ are independently substituted with D, R, K, G, A, V or S, optionally provided that at least one of X₁₆₁, X₁₆₂, X₁₆₃ and X₁₆₄ is an amino acid not found in SEQ ID NOS: 1 or 3. In another embodiment, X₃₉ is D, X₄₀ is K, one of X₇₆ and X₈₀ is A or S and the other of X₇₆ and X₈₀ is C and X₁₆₁, X₁₆₂, X₁₆₃ and X₁₆₄ are selected from the combinations shown in Table 2. In still another embodiment, X₃₉ is D, X₄₀ is K, one of X₇₆ and X₈₀ is A or S and the other of X₇₆ and X₈₀ is C and X₁₆₁, X₁₆₂, X₁₆₃ and X₁₆₄ are GSGS or GAAG.

In one embodiment, the Angptl4 derivative is based on a fragment of Angplt4. Suitable fragments include any fragment that retains the activity of wild type Angplt4 or any fragment of 100 or more consecutive amino acids. In one embodiment, such fragment is based on amino acids 1-187 SEQ ID NO: 1 or amino acids 1-182 of SEQ ID NO: 3. Such fragments may have the amino acid substitutions described in the preceding paragraphs.

The Angptl4 polypeptide derivative may have an activity that is comparable to or increased (in one embodiment, 50% or more) as compared to the wild-type Angptl4 polypeptide activity; alternatively, the Angptl4 polypeptide derivative may have an activity that is decreased (in one embodiment, less than 50%) as compared to the wild-type Angptl4 polypeptide activity. In a specific embodiment, the Angptl4 polypeptide derivative has a decreased ability to inhibit LPL and shows an increased resistance to cleavage.

The deletions, additions and substitutions can be selected, as would be known to one of ordinary skill in the art, to generate a desired Angptl4 polypeptide derivative. For example, conservative substitutions or substitutions of amino acids with similar properties are expected to be tolerated. In addition, specific deletions, insertions and substitutions may impact, positively or negatively, a certain Angptl4 polypeptide activity but not impact a different Angptl4 polypeptide activity.

Conservative modifications to the amino acid sequence of any of SEQ ID NOS: 1 or 3 or 5 or 7, including combinations thereof (and the corresponding modifications to the encoding nucleotides) will produce Angptl4 polypeptide derivatives having functional and chemical characteristics similar to those of naturally occurring Angptl4 polypeptides while minimizing undesirable properties such as LPL inhibitory activity. In contrast, substantial modifications in the functional and/or chemical characteristics of Angptl4 polypeptides may be accomplished by selecting substitutions in the amino acid sequence of any of SEQ ID NOS: 1 or 3 or 5 or 7, including combinations thereof, that differ significantly in their effect on maintaining (a) the structure of the molecular backbone in the area of the substitution.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine.

Conservative amino acid substitutions also encompass non-naturally occurring amino acid residues which are typically incorporated by chemical peptide synthesis rather than by synthesis in biological systems. These include peptidomimetics, and other reversed or inverted forms of amino acid moieties. It will be appreciated by those of skill in the art that nucleic acid and polypeptide molecules described herein may be chemically synthesized as well as produced by recombinant means.

Naturally occurring residues may be divided into classes based on common side chain properties: 1) hydrophobic: norleucine, Met, Ala, Val, Leu, Ile; 2) neutral hydrophilic: Cys, Ser, Thr, Asn, Gln; 3) acidic: Asp, Glu; 4) basic: His, Lys, Arg; 5) residues that influence chain orientation: Gly, Pro; and 6) aromatic: Trp, Tyr, Phe.

For example, non-conservative substitutions may involve the exchange of a member of one of these classes for a member from another class. Such substituted residues may be introduced into regions of the Angptl4 polypeptide derivatives that are homologous with non-human Angptl4 polypeptide orthologs, or into the non-homologous regions of the molecule.

In making such changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art (Kyte et al., J. Mol. Biol., 157:105-131, 1982). It is known that certain amino acids may be substituted for other amino acids having a similar hydropathic index or score and still retain a similar biological activity.

In making changes based upon the hydropathic index, the substitution of amino acids whose hydropathic indices are within +/- 2 may be used; in an alternate embodiment, the hydropathic indices are with +/- 1; in yet another alternate embodiment, the hydropathic indices are within +/- 0.5.

It is also understood in the art that the substitution of like amino acids can be made effectively on the basis of hydrophilicity. The greatest local average hydrophilicity of a polypeptide as governed by the hydrophilicity of its adjacent amino acids, correlates with a biological property of the protein.

The following hydrophilicity values have been assigned to amino acid residues: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making changes based upon similar hydrophilicity values, the substitution of amino acids whose hydrophilicity values are within +/- 2 may be used; in an alternate embodiment, the hydrophilicity values are with +/- 1; in yet another alternate embodiment, the hydrophilicity values are within +/- 0.5.

Desired amino acid substitutions (whether conservative or non-conservative) can be determined by those skilled in the art at the time such substitutions are desired. For example, amino acid substitutions can be used to identify important residues of the Angptl4 polypeptide, or to increase or decrease the affinity of the Angptl4 polypeptide with a particular binding target in order to increase or decrease an Angptl4 polypeptide activity.

Exemplary amino acid substitutions are set forth in Table 3.

| Table 3 | | |
|---|---|---|
| Original Amino Acid | Exemplary substitution | Preferred substitution |
| Ala | Val, Leu, Ile | Val |
| Arg | Lys, Gln, Asn | Lys |
| Asn | Glu | Glu |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Ile, Val, Met, Ala, Phe, Norleucine | Ile |
| Lys | Arg, 1,4-diaminobutyric acid, Gln, Asn | Arg |
| Met | Leu, Phe, Ile | Leu |
| Phe | Leu, Val, Ile, Ala, Tyr | Leu |
| Pro | Ala, Gly | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of the polypeptide as set forth in any of SEQ ID NOS: 1 0r 3 or 5 or 7, including combinations thereof, using well known techniques. For identifying suitable areas of the molecule that may be changed without destroying activity, one skilled in the art may target areas not believed to be important for activity. For example, when similar polypeptides with similar activities from the same species or from other species are known, one skilled in the art may compare the amino acid sequence of an Angptl4 polypeptide to such similar polypeptides. With such a comparison, one can identify residues and portions of the molecules that are conserved among similar polypeptides. It will be appreciated that changes in areas of an Angptl4 polypeptide that are not conserved relative to such similar polypeptides would be less likely to adversely affect the biological activity and/or structure of the Angptl4 polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in an Angptl4 polypeptide that correspond to amino acid residues that are important for activity or structure in similar polypeptides. One skilled in the art may opt for chemically similar amino acid substitutions for such predicted important amino acid residues of anAngptl4 polypeptide.

One skilled in the art can also analyze the three-dimensional structure and amino acid sequence in relation to that structure in similar polypeptides. In view of that information, one skilled in the art may predict the alignment of amino acid residues of an Angptl4 polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test Angptl4 polypeptide derivatives containing a single amino acid substitution at each desired amino acid residue. The derivatives can then be screened using activity assays know to those skilled in the art and as disclosed herein. Such derivatives could be used to gather information about suitable substitution. For example, if one discovered that a change to a particular amino acid residue resulted in destroyed, undesirably reduced, or unsuitable activity, derivatives with such a change would be avoided. In other words, based on information gathered from such routine experiments, one skilled in the art can readily determine the amino acids where further substitutions should be avoided either alone or in combination with other mutations.

Numerous scientific publications have been devoted to the prediction of secondary structure from analyses of amino acid sequences (see Chou et al., Biochemistry, 13(2):222-245, 1974; Chou et al., Biochemistry, 113(2):211-222, 1974; Chou et al., Adv. Enzymol. Relat. Areas Mol. Biol., 47:45-148, 1978; Chou et al., Ann. Rev. Biochem., 47:251-276, 1979; and Chou et al., Biophys. J., 26:367-384, 1979). Moreover, computer programs are currently available to assist with predicting secondary structure of polypeptides. Examples include those programs based upon the Jameson-Wolf analysis (Jameson et al., Comput. Appl. Biosci., 4(1):181-186, 1998; and Wolf et al., Comput. Appl. Biosci., 4(1):187-191; 1988), the program PepPlot.RTM. (Brutlag et al., CABS, 6:237-245, 1990; and Weinberger et al., Science, 228:740-742, 1985), and other new programs for protein tertiary structure prediction (Fetrow. et al., Biotechnology, 11:479-483, 1993).

Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural data base (PDB) has provided enhanced predictability of secondary structure, including the potential number of folds within a polypeptide's or protein's structure (see Holm et al., Nucl. Acid. Res., 27(1):244-247, 1999).

Additional methods of predicting secondary structure include "threading" (Jones, D., Curr. Opin. Struct. Biol., 7(3):377-87, 1997; Suppl et al., Structure, 4(1):15-9, 1996), "profile analysis" (Bowie et al., Science, 253:164-170, 1991; Gribskov et al., Meth. Enzym., 183:146-159, 1990; and Gribskov et al., Proc. Nat. Acad. Sci., 84(13): 4355-4358, 1987), and. "evolutionary linkage" (See Home, supra, and Brenner, supra).

Any of the polypeptide forms discussed herein may also contain a sequence useful in the identification or purification of the polypeptide; an example of such a sequence is the C-terminal V5 tag. The foregoing also includes nucleic acid sequences (such as, but not limited to cDNA sequences) coding for such polypeptides, including polypeptide derivatives as described herein.

### Compositions

Useful compositions of the present disclosure may comprise one or more polypeptides of the present disclosure useful in the treatment and prevention methods of the present disclosure; useful compositions also include one or more nucleic acids coding for one or more polypeptides of the present disclosure useful in the treatment and prevention methods of the present disclosure. The compositions disclosed may comprise one or more of such compounds, in combination with a pharmaceutically acceptable carrier. Examples of such carriers and methods of formulation may be found in Remington: The Science and Practice of Pharmacy (20th Ed., Lippincott, Williams & Wilkins, Daniel Limmer, editor). To form a pharmaceutically acceptable composition suitable for administration, such compositions will contain an therapeutically effective amount of compound.

The pharmaceutical compositions of the disclosure may be used in the treatment and prevention methods of the present disclosure. Such compositions are administered to a subject in amounts sufficient to deliver a therapeutically effective amount of the compound(s) so as to be effective in the treatment and prevention methods disclosed herein. The therapeutically effective amount may vary according to a variety of factors such as, but not limited to, the subject's condition, weight, sex and age. Other factors include the mode and site of administration. The pharmaceutical compositions may be provided to the subject in any method known in the art. Exemplary routes of administration include, but are not limited to, subcutaneous, intravenous, topical, epicutaneous, oral, intraosseous, and intramuscular. The compositions of the present disclosure may be administered only one time to the subject or more than one time to the subject. Furthermore, when the compositions are administered to the subject more than once, a variety of regimens may be used, such as, but not limited to, one per day, once per week or once per month. The compositions may also be administered to the subject more than one time per day. The therapeutically effective amount and appropriate dosing regimens may be identified by routine testing in order to obtain optimal activity, while minimizing any potential side effects. In addition, co-administration or sequential administration of other agents may be desirable.

The compositions of the present disclosure may be administered systemically, such as by intravenous administration, or locally such as by subcutaneous injection or by application of a paste or cream.

In one embodiment, a nucleic acid, which may be in the form of a suitable plasmid or vector, is provided that codes for an Angptl4 polypeptide or Angptl4 polypeptide variant of the present disclosure. Such nucleic acid is introduced into a cell, which may be obtained from the subject, by suitable methods known in the art (for example, electroporation). In one embodiment, the cell is an adipose cell. The cells may be assayed for expression of the Angptl4 polypeptide or polypeptide derivative (in one embodiment, expression of the polypeptide can be determined by the presence of a tag on the polypeptide as discussed herein). The cells expressing an Angptl4 polypeptide of polypeptide derivative may then be introduced into the subject. In one embodiment, the cells are administered to the subject by subcutaneous injection; other methods of administration may also be used, including those discussed herein. The cells then express Angptl4 polypeptide or an Angptl4 polypeptide derivative, which is taken up into the circulation.

The compositions of the present disclosure may further comprise agents which improve the solubility, half-life, absorption, etc. of the compound(s). Furthermore, the compositions of the present disclosure may further comprise agents that attenuate undesirable side effects and/or or decrease the toxicity of the compounds(s). Examples of such agents are described in a variety of texts, such a, but not limited to, Remington: The Science and Practice of Pharmacy (20th Ed., Lippincott, Williams & Wilkins, Daniel Limmer, editor).

The compositions of the present disclosure can be administered in a wide variety of dosage forms for administration. For example, the compositions can be administered in forms, such as, but not limited to, tablets, capsules, sachets, lozenges, troches, pills, powders, granules, elixirs, tinctures, solutions, suspensions, elixirs, syrups, ointments, creams, pastes, emulsions, or solutions for intravenous administration or injection. Other dosage forms include administration transdermally, via patch mechanism or ointment. Any of the foregoing may be modified to provide for timed release and/or sustained release formulations.

In the present disclosure, the pharmaceutical compositions may further comprise a pharmaceutically acceptable carriers include, but are not limited to, vehicles, adjuvants, surfactants, suspending agents, emulsifying agents, inert fillers, diluents, excipients, wetting agents, binders, lubricants, buffering agents, disintegrating agents and carriers, as well as accessory agents, such as, but not limited to, coloring agents and flavoring agents (collectively referred to herein as a carrier). Typically, the pharmaceutically acceptable carrier is chemically inert to the active compounds and has no detrimental side effects or toxicity under the conditions of use. The pharmaceutically acceptable carriers can include polymers and polymer matrices. The nature of the pharmaceutically acceptable carrier may differ depending on the particular dosage form employed and other characteristics of the composition.

For instance, for oral administration in solid form, such as but not limited to, tablets, capsules, sachets, lozenges, troches, pills, powders, or granules, the compound(s) may be combined with an oral, non-toxic pharmaceutically acceptable inert carrier, such as, but not limited to, inert fillers, suitable binders, lubricants, disintegrating agents and accessory agents. Suitable binders include, without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, and the like. Disintegrators include, without limitation, starch, methyl cellulose, agar, bentonite, xanthum gum and the like. Tablet forms can include one or more of the following: lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid as well as the other carriers described herein. Lozenge forms can comprise the active ingredient in a flavor, usually sucrose and acacia or tragacanth, as well as pastilles comprising the active ingredient in an inert base, such as gelatin and glycerin, or sucrose and acadia, emulsions, and gels containing, in addition to the active ingredient, such carriers as are known in the art.

For oral liquid forms, such as but not limited to, tinctures, solutions, suspensions, elixirs, syrups, the nucleic acid molecules of the present disclosure can be dissolved in diluents, such as water, saline, or alcohols. Furthermore, the oral liquid forms may comprise suitably flavored suspending or dispersing agents such as the synthetic and natural gums, for example, tragacanth, acacia, methylcellulose and the like. Moreover, when desired or necessary, suitable and coloring agents or other accessory agents can also be incorporated into the mixture. Other dispersing agents that may be employed include glycerin and the like.

Formulations suitable for parenteral administration include aqueous and non-aqueous, isotonic sterile injection solutions, which can contain anti-oxidants, buffers, bacteriostats, and solutes that render the formulation isotonic with the blood of the patient, and aqueous and non-aqueous sterile suspensions that can include suspending agents, solubilizers, thickening agents, stabilizers, and preservatives. The compound(s) may be administered in a physiologically acceptable diluent, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol such as poly(ethyleneglycol) 400, glycerol ketals, such as 2,2-dimethyl-1,3-dioxolane-4-methanol, ethers, an oil, a fatty acid, a fatty acid ester or glyceride, or an acetylated fatty acid glyceride with or without the addition of a pharmaceutically acceptable surfactant, such as, but not limited to, a soap, an oil or a detergent, suspending agent, such as, but not limited to, pectin, carbomers, methylcellulose, hydroxypropylmethylcellulose, or carboxymethylcellulose, or emulsifying agents and other pharmaceutical adjuvants.

Oils, which can be used in parenteral formulations, include petroleum, animal, vegetable, or synthetic oils. Specific examples of oils include peanut, soybean, sesame, cottonseed, corn, olive, petrolatum, and mineral. Suitable fatty acids for use in parenteral formulations include polyethylene sorbitan fatty acid esters, such as sorbitan monooleate and the high molecular weight adducts of ethylene oxide with a hydrophobic base, formed by the condensation of propylene oxide with propylene glycol, oleic acid, stearic acid, and isostearic acid. Ethyl oleate and isopropyl myristate are examples of suitable fatty acid esters. Suitable soaps for use in parenteral formulations include fatty alkali metal, ammonium, and triethanolamine salts, and suitable detergents include (a) cationic detergents such as, for example, dimethyldialkylammonium halides, and alkylpyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene polypropylene copolymers, (d) amphoteric detergents such as, for example, alkylbeta-aminopropionates, and 2-alkylimidazoline quaternary ammonium salts, and (e) mixtures thereof.

Suitable preservatives and buffers can be used in such formulations. In order to minimize or eliminate irritation at the site of injection, such compositions may contain one or more nonionic surfactants having a hydrophile-lipophile balance (HLB) of from about 12 to about 17. The quantity of surfactant in such formulations ranges from about 5% to about 15% by weight.

Topical dosage forms, such as, but not limited to, ointments, creams, pastes, emulsions, containing the nucleic acid molecule of the present disclosure, can be admixed with a variety of carrier materials well known in the art, such as, e.g., alcohols, aloe vera gel, allantoin, glycerine, vitamin A and E oils, mineral oil, PPG2 myristyl propionate, and the like, to form alcoholic solutions, topical cleansers, cleansing creams, skin gels, skin lotions, and shampoos in cream or gel formulations. Inclusion of a skin exfoliant or dermal abrasive preparation may also be used. Such topical preparations may be applied to a patch, bandage or dressing for transdermal delivery or may be applied to a bandage or dressing for delivery directly to the site of a wound or cutaneous injury.

The compound(s) of the present disclosure can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines. Such liposomes may also contain monoclonal antibodies to direct delivery of the liposome to a particular cell type or group of cell types.

The compound(s) of the present disclosure may also be coupled with soluble polymers as targetable drug carriers. Such polymers can include, but are not limited to, polyvinyl-pyrrolidone, pyran copolymer, polyhydroxypropylmethacryl-amidephenol, polyhydroxyethylaspartamidephenol, or polyethyl-eneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

### RESULTS

In the following results, the methods used were those methods specified in the Methods section of the present disclosure and the references cited therein. Some of the following results are described in Clement LC et. al., Podocyte secreted Angiopoietin-like 4 mediates proteinuria in glucocorticoid-sensitive nephrotic syndrome, Nature Medicine, January 2011(this reference is hereby incorporated by reference for the disclosure contained therein regarding the use of Angptl4 polypeptides).

### Patients with Nephrotic Syndrome Have Increased Levels of Circulating Angptl4

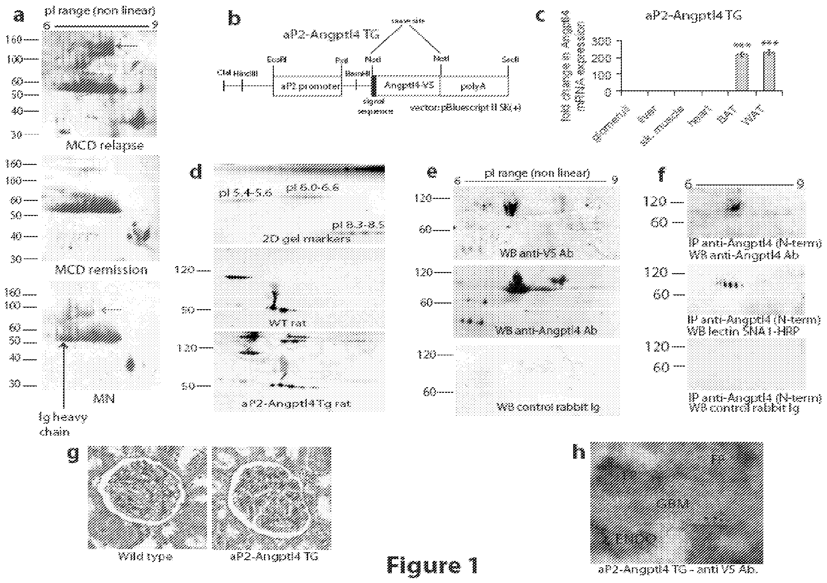

Patients with Nephrotic syndrome have increased circulating levels of Angptl4 polypeptide. 200 *µ*g human plasma from patients (n = 4 patients/group) with diagnosed with MCD and MN and patients in MCD relapse were analyzed by 2D gel electrophoresis and Western blots were prepared using anti-Angptl4 antibodies (FIG. 1A). FIG. 1A shows that only patients with MCD relapse and MN had increased levels of Angptl4 (indicated by arrows). This form of Angptl4 exists as a neutral pI form and is present as monomers and oligomers.

### aP2-Angptl4 TG Rats Have Increased Circulating Levels of Angptl4

A transgenic rat models for adipocyte specific Angptl4 overexpression was developed and is shown in FIG. 1B (aP2-Angptl4 TG). Analysis of mRNA expression in organs that normally express Angptl4 confirmed specificity of expression, with Angplt4 being detected in brown adipose tissue (BAT) and white adipose tissue (WAT) (FIG. 1C).

2D gel electrophoresis of 200 *µ*g plasma, followed by Western blotting using an anti-Angptl4 antibody revealed that heterozygous aP2-Angptl4 TG rats had higher circulating Angptl4 levels than wild type rats (FIG. 1D) (age 3 months, n = 3 blots/group). FIG. 1E shows 2D gel electrophoresis of 200 *µ*g plasma, followed by Western blotting using anti-Angptl4 and anti-V5 antibodies show the presence of adipose tissue secreted V5-tagged Angptl4 in the plasma of aP2-Angptl4 TG rats. 2D gel electrophoresis of immunoprecipitated Angptl4 from aP2-Angptl4 TG rat plasma (using an antibody specific for the N-terminus of Angptl4), followed by Western blotting using anti-Angptl4 or anti-lectin. SNA I antibodies revealed the presence of sialylated Angptl4 polypeptide in the circulation.

The aP2-Angptl4 TG rats had morphologically normal glomeruli by light (FIG. 1G) and electron microscopy (not shown), and glomerular Angptl4 expression was unchanged. This is in contrast to podocyte specific expression of Angptl4, where such expression resulted in glomerular defects, including progressive development of foot process effacement between age one to five months (see U.S. Provisional application No. 61/351,865 (filed 5 June 2010), which is hereby incorproated by reference for such teaching).

Immunogold EM using anti-V5 antibody to specifically detect transgene expressed protein in 3 month old heterozygous aP2-Angptl4 TG male rats demonstrated detection selectively on the endothelial surface, indicating that circulating Angptl4 middle and high order oligomers do not enter the GBM and have receptors on the endothelial surface. The effects of circulating Angptl4 is relevant to both human and experimental nephrotic syndrome, since adipose tissue upregulation of Angptl4 is noted in later stages of nephrotic syndrome, when proteinuria is on the decline.

### Relationship of Increased Circulating Levels of Angptl4 with Proteinuria and Albuminuria

To examine the relationship between circulating levels of Angptl4 proteinuria, including albuminuria, proteinuria was analyzed in aP2-Angptl4 TG rats. FIG. 2A shows that that aP2-Angptl4 TG do not exhibit proteinuria as determined by analysis of urinary proteins. In FIG. 2A urinary proteins were analyzed by GelCode blue stained SDS PAGE (3 *µ*g / lane, except MCD remission) (densitometry readings are provided under each lane). The intact albumin band is observed at 70 kDa (indicated by arrow). As can be seen, WT rats, aP2-Angptl4 TG rats and MCD patients in remission showed little or no intact albumin in the analysed urinary samples, wherein NPHS2-Angplt4 TG rats (a rat transgenic model having podocytes specific Angptl4 expression and shown to develop MCD with proteinuria; see US Provisional application No. , which is hereby incorproated by reference for such teaching), MCD relapse, MN relapse and PAN rats (a rat model of nephrotic syndrome) showed strong albumin staining indicative of albuminuria. FIG. 2B shows that female heterozygous aP2-Angptl4 female TG rats had decreased albuminuria as compared to WT littermate controls. FIG. 2C shows the same results for aP2-Angptl4 heterozygous male TG rats. FIG. 2D shows that aP2-Angptl4 TG rats exhibited reduced proteinuria in the puromycin nephrosis (PAN model; a rat model of nephrotic syndrome) as compared to WT littermates. As demonstrated above, aP2-Angptl4 TG rats have higher circulating Angptl4 levels that migrate at or around neutral isoelectric point, and is sialylated. These results show a role for circulating Angptl4 in reducing proteinuria and nephrotic syndrome.

Since endothelial binding of adipose tissue secreted Angptl4 bound to glomerular endothelium, experiments were conducted to determine the effect of recombinant Angptl4 on glomerular epithelial cells (GEnCs) to investigate whether lower baseline albuminuria and less PAN induced proteinuria in this rat model were mediated by glomerular endothelial protection. GEnCs were subject to oxidative injury by addition of hydrogen peroxide and into the culture media and incubated with concentrated supernatant (600 *µ*g / well) from the control stable cell line, Angptl4-HEK293 cell line (secreting high isoelectric point (pI), hyposialylated Angptl4) or Angptl4-HEK293 cell line incubated with ManNAc (neutral pI, normally sialylated Angptl4). It should be noted that the high pI form of Angptl4 is secreted in large amounts from podocytes in MCD. Release of LDH was assessed as a marker of cell injury. Control cells without hydrogen peroxide injury were given a relative score of 1. High pI Angptl4 increased GEnC injury, whereas neutral pI Angptl4 (which comprises most of circulating Angptl4) was significantly protective at all measured time points. (n = 3 readings/condition).

Upregulation of Angptl4 in wild type rats on PAN Day 6 was exclusively glomerular, whereas upregulation in adipose tissue was noted on Day 10 when proteinuria and glomerular Angptl4 expression are on the decline (n = 3 rats / sample) (FIG. 2F). Therefore, increases in circulating Angptl4 levels are coincident with the protective effect of circulating Angptl4 in nephrotic syndrome and reduction of proteinuria. The effects of circulating Angptl4 are likely to be relevant to both human and experimental MCD, since adipose tissue upregulation of Angptl4 is noted in later stages of PAN when proteinuria is on the decline. Furthermore, increased circulating Angptl4 levels at baseline and after induction of PAN in aP2-Angptl4 TG rats resulted in increased plasma triglyceride levels (FIG. 2G) and reduced post-heparin lipoprotein lipase activity (FIG. 2H) as compared to wild type rat.

In order to demonstrate the effectiveness of the therapeutic delivery of Angptl4 into the circulation, wild type Angptl4 or a control protein was administered to Buffalo/Mna rats, a model of FSGS, or to Wistar rats in which Thy1.1 nephritis, a short term model of mesangial injury, was induced (FIGS. 4A and B). Wild-type recombinant Angptl4 polypeptide was generated by harvesting of recombinant protein. *Angptl4*-HEK293 stable or pcDNA3.1-HEK293 control stable cell lines were grown to confluence in 15 cm dishes, washed twice with warm PBS, and incubated with serum free DMEM without Phenol Red, with or without 25 mM ManNAc, for 48 hours. Cells were harvested and the supernatant concentrated. Concentrated supernatant from one 15 cm dish was used at each injection time point.

Buffalo/Mna rats spontaneously develop lesions mimicking human FSGS at around age 2 months, including focal and segmental lesions on light microscopy, effacement of podocyte foot processes on electron microscopy, and proteinuria. The rats develop progressive increase in proteinuria as they age. The rats used in the above studies were male and 5 months old. Anti-Thy1.1 nephritis was induced by injection of 150 *µ*g of anti-Thy1.1 (Ox-7 hybridoma) or control IgG IV into different groups of male Wistar rats (100-125 gm, n = 4 rats/group).

In the Buffalo/Mna rat model, assessment of baseline proteinuria was made on Day 0. Angptl4 or control protein were injected intra-peritoneally on two consecutive days (Days 1 & 2, arrows) into Buffalo Mna rats (n=4 rats/group). Proteinuria was assessed on alternate days, and expressed as a percentage of baseline values. Significant reduction in proteinuria was noted in recombinant Angptl4 treated rats.

In theThy1.1 nephritis model, proteinuria confirmed on Day 1. Rats were injected intravenously with either recombinant Angptl4 or control protein on two consecutive days (Days 1 & 2, arrows). Proteinuria was then assessed. As shown in FIG. 4B, proteinuria was lower in Angptl4 treated rats throughout, and was statistically significant on Day 5.

These results show that therapeutic delivery of Angptl4 into the circulation are an effective treatment for nephrotic syndrome, such as but not limited to minimal change disease, focal segmental glomerulosclerosis, membranous nephropathy/membranous glomerulonephritis, membranoproliferative glomerulonephritis or a diabetic condition, such as, but not limited to, diabetic nephropathy, diabetes mellitus, lupus nephritis or primary glomerular disease. Furthermore, these results show that therapeutic delivery of Angptl4 into the circulation are an effective treatment for and conditions related to nephrotic syndrome, such as but not limited to, proteinuria, hypercholesterolemia, hypertriglyceridemia and edema. In one embodiment, the Angptl4 polypeptide is a derivative with decreased LPL inhibitory activity, resistance to cleavage or a derivative described herein. Administration of such a derivative would retain the beneficial effects of Angptl4 treatment without the negative effects associated with inhibition of LPL activity, such as increased plasma triglyceride levels.

### METHODS

### Cloning of full length rat Angptl4, and generation of antibody against full length recombinant Angptl4

The full length rat Angptl4 open reading frame of 1218 bp from our previous experiments (7), excluding the stop codon, was cloned into pcDNA3.1/V5-HisB for eukaryotic expression, and into pET28a for prokaryotic expression. The E. Coli expressed purified full length protein was used to generate a polyclonal antibody in rabbits (Proteintech group, Inc. Chicago IL USA) that was tested by ELISA and Western blot. Antibody reactive bands were excised from GelCode blue stained gels, trypsin digested and presence of Angptl4 peptide sequences confirmed by MALDI-TOF/TOF. Part of the antiserum was affinity purified to the antigen. Unless otherwise specified, all studies described used this antibody. An additional polyclonal antibody against the N-terminal part of rat Angptl4 (amino acids 7 - 86 excluding signal peptide) was similarly raised in rabbits.

### Induction of proteinuria in animal models of human glomerular disease

All animal studies were approved by the institutional IACUC. Induction of animal models of proteinuria (n = 4 rats/group) in WT rats are described in previous publications in parenthesis: PAN (7), PHN (7), PAN with glucocorticoids (20), non-HIV collapsing glomerulopathy (18), nephrotoxic serum induced heterologous phase proteinuria (7). Anti-Thy1.1 nephritis was induced by injection of 200 mcg of anti-Thy1.1 (Ox-7 hybridoma) or control IgG IV into different groups of male Wistar rats (100-125 gm, n = 4 rats/group), and rats euthanized after 24 and 72 hours.

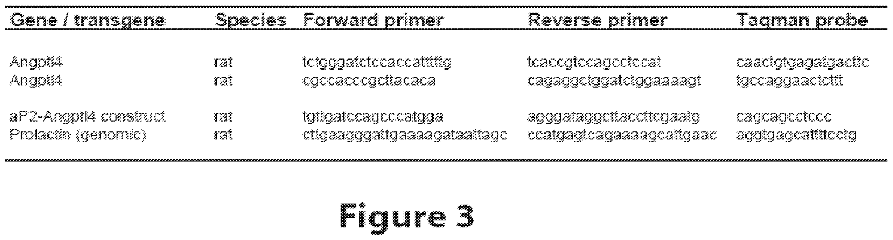

The following techniques are described in prior publications: Taqman real time PCR (26), confocal imaging (7), in situ hybridization (27), immunogold EM (26), glomerular extraction and processing for Western blot (26), assessment of charge by PEI method (28). For alcian blue staining, the pH of the staining solution was adjusted to 2.5 using acetic acid, and 0.1% nuclear fast red solution was used as a counterstain. Densitometry of glomerular basement membrane alcian blue stain (20 glomeruli/rat, 3 rats/group) was assessed using Image-Pro software (Media Cybernetics, Inc., Bethesda MD, USA). Densitometry of 2D gel Western blots was assessed using Gel-Pro Analyzer software (Media Cybernetics, Inc.). Taqman real time PCR primers and probes are listed in FIG. 3. For *in situ* hybridization, the digoxigenin labeled probe for rat Angptl4 included bp 1 to 548 of the ORF.

To obtain samples for post heparin LPL activity, rats were injected intravenously with 10 units/100 gm weight of porcine heparin 15 minutes prior to euthanasia, and activity measured using an assay from Roar Biomedical, Inc (New York NY). Serum triglycerides were measured in the fasting state.

### Injection of NTS into Angptl4 -/- mice

Angptl4 -/- mice were provided to Sander Kersten as a kind gift from Eli Lily Corporation (Indianapolis IN USA). The study protocol was approved by the Animal Studies Committee at Wageningen University. Eleven week old male Angptl4 -/- or +/+ mice (n = 4 mice/group) were injected intravenously with 1.5 mg γ2-NTS or normal sheep serum (Sigma Aldrich St. Louis MO USA), spot urine samples collected at 48 hours, mice euthanized at 72 hours, plasma collected for biochemical measurements, and kidneys preserved for histological analysis. Urine albumin was assessed by ELISA (Bethyl laboratories, Montgomery TX USA) and urine creatinine measured by mass spectrometry. To assess for foot process effacement, the mean width of foot processes was first measured in control treated Angptl4 +/+ mouse transmission electron micrographs (10 equally spaced readings/loop, 3 loops/glomerulus, 3 glomeruli/kidney, 3 kidneys/group). Effacement was described as an over 2.5 fold increase in mean width. Total and effaced foot processes were counted in NTS treated or control treated Angptl4 -/- mice.

### Studies with archived human samples

Immunostaining of archived human kidney biopsies (n = 5 biopsies per condition) was conducted on samples obtained via IRB approved protocols at the Instituto Nacional de Cardiologia, Mexico City. Control kidney biopsies used for these studies were sex and age matched protocol pre-transplant biopsies. Archival human sera for 2D gel electrophoresis and Western blot (n = 4 samples / condition) were obtained from a previously published study (29).

### Generation of transgenic rats

aP2-Angptl4 TG rats (adipose tissue specific) construct was generated in the vector that contained the 5.4 Kb mouse aP2 promoter construct (30) (purchased from Addgene Inc. Cambridge MA USA) by cloning the rat Angptl4 cDNA (including the signal sequence) with a C-terminal V5 tag at the NotI site just upstream of the polyA tail.

Transgenic rats were generated by microinjection of the digested DNA constructs into fertilized Sprague Dawley eggs (conducted at University of Michigan), implantation into pseudopregnant host Sprague Dawley females, and the resulting offsprings were genotyped by routine PCR and TaqMan genomic DNA real time PCR strategy using construct specific and control genomic prolactin primer and probe combinations (FIG. 3). Three founder lines for adipose tissue specific expression were generated. Data from aP2-Angptl4 TG rat line 375 (3 copies), both stable over 4 generations, are presented. Urinary total protein was assessed using the Bradford method (Biorad laboratories, Hercules CA USA), and albuminuria by ELISA (Bethyl laboratories, Montgomery TX USA).

### In vitro studies with GEnCs

For GEnC studies, cultured rat GEnCs (32) were grown to 75% confluence in 6 well plates (n = 3 wells / condition), washed twice with warm PBS, serum free RPMI containing 200 *µ*M H2O2, along with 600 *µ*g / well of control stable cell line supernatant, or Angptl4-HEK293 stable cell line supernatant, or supernatant from ManNAc treated Angptl4-HEK293 cell line. Wells were sampled at 24, 36 and 48 hours. LDH release was measured using the cytotoxicity detection kit (Roche Diagnostics, Mannheim Germany). OD 492 values were expressed as a ratio of readings from wells in which no H2O2 or stable cell line supernatant was added.

### Statistical analysis

Analysis of difference in proteinuria or gene expression involving three or more groups was conducted by ANOVA with post analysis testing using GraphPad InStat software, Version 3.05. For comparison of two groups, the unpaired Students t test in Microsoft Excel 2003 was used.

### REFERENCES

1. Falk R, Jennette C, Nachman PH. Primary glomerular disease. In The Kidney, Brenner BM, editor, 6th edition, 1263-1349 (2000).
2. Gutman, A. & Shafrir, E. Adipose tissue in experimental nephrotic syndrome. Am. J. Physiol. 205, 702-706 (1963).
3. Vaziri, N.D. Molecular mechanisms of lipid disorders in nephrotic syndrome. Kidney Int. 63, 1964-1976 (2003).
4. Shearer, G.C. & Kaysen GA. Endothelial bound lipoprotein lipase (LpL) depletion in hypoalbuminemia results from decreased endothelial binding, not decreased secretion. Kidney Int. 70, 647-653 (2006).
5. Reaven, E.P., Kolterman, O.G. & Reaven, G.M. Ultrastructural and physiological evidence for corticosteroid-induced alterations in hepatic production of very low density lipoprotein particles. J. Lipid Res. 15, 74-83 (1974).
6. Tsukamoto, Y., Kokubo, T., Horii, A., Moriya, R. & Kobayashi, Y. Lipoprotein derangement during steroid treatment in minimal-change nephrotic syndrome. Nephron 73, 606-612 (1996).
7. Liu, G., Clement, L., Kanwar, Y.S., Avila-Casado, C. & Chugh, S.S. ZHX proteins regulate podocyte gene expression during the development of nephrotic syndrome. J. Biol. Chem. 281, 39681-39692 (2006*).*
8. Yoon, J.C. et al. Peroxisome proliferator-activated receptor gamma target gene encoding a novel angiopoietin-related protein associated with adipose differentiation. Mol. Cell. Biol. 20, 5343-5349 (2000).
9. Kersten, S. et al. Characterization of the fasting-induced adipose factor FIAF, a novel peroxisome proliferator-activated receptor target gene. J. Biol. Chem. 275, 28488-28493 (2000).
10. Kim, I. et al. Hepatic expression, synthesis and secretion of a novel fibrinogen/angiopoietin-related protein that prevents endothelial-cell apoptosis. Biochem. J. 346, 603-610 (2000).
11. Yoshida, K., Shimizugawa, T., Ono, M. & Furukawa, H. Angiopoietin-like protein 4 is a potent hyperlipidemia-inducing factor in mice and inhibitor of lipoprotein lipase. J. Lipid Res. 43, 1770-1772 (2002).
12. Ge, H., et al. Oligomerization and regulated proteolytic processing of angiopoietin-like protein 4. J. Biol. Chem. 279, 2038-2045 (2004).
13. Ge, H., Yang, G., Yu, X., Pourbahrami, T. & Li, C. Oligomerization state-dependent hyperlipidemic effect of angiopoietin-like protein 4. J. Lipid Res. 45, 2071-2079 (2004).
14. Romeo, S., et al. Population-based resequencing of ANGPTL4 uncovers variations that reduce triglycerides and increase HDL. Nat. Genet. 39, 513-516 (2007).
15. Romeo, S. et al. Rare loss-of-function mutations in ANGPTL family members contribute to plasma triglyceride levels in humans. J. Clin. Invest. 119:70-79 (2009).
16. Eremina, V., et al. VEGF inhibition and renal thrombotic microangiopathy. N. Engl. J. Med. 358, 1129-1136 (2008).
17. Davis, B., et al. Podocyte-specific expression of angiopoietin-2 causes proteinuria and apoptosis of glomerular endothelia. J. Am. Soc. Nephrol. 18, 2320-2329 (2007).
18. Avila-Casado, C., et al. Proteinuria in rats induced by serum from patients with collapsing glomerulopathy. Kidney Int. 66, 133-143 (2004).
19. Mandard, S., et al. The fasting-induced adipose factor/angiopoietin-like protein 4 is physically associated with lipoproteins and governs plasma lipid levels and adiposity. J. Biol. Chem. 281:934-944 (2006).
20. Clement, L., et al. Early changes in gene expression that influence the course of primary glomerular disease. Kidney Int. 72, 337-347 (2007).
21. Cazes, A. et al. Extracellular matrix-bound angiopoietin-like 4 inhibits endothelial cell adhesion, migration, and sprouting and alters actin cytoskeleton. Circ. Res. 99, 1207-1215 (2006).
22. Malicdan, M.C., Noguchi, S., Hayashi, Y.K., Nonaka, I. & Nishino, I. Prophylactic treatment with sialic acid metabolites precludes the development of the myopathic phenotype in the DMRV-hIBM mouse model. Nat. Med. 15, 690-695 (2009).
23. Galeano, B. et al. Mutation in the key enzyme of sialic acid biosynthesis causes severe glomerular proteinuria and is rescued by N-acetylmannosamine. J. Clin. Invest. 117, 1585-1594 (2007).
24. Ruge, T. et al. Lipoprotein lipase in the kidney: activity varies widely among animal species. Am. J. Physiol. Renal Physiol. 287, F1131-F1139 (2004).
25. Koliwad, S.K. et al. Angiopoietin-like 4 (ANGPTL4/FIAF) is a direct glucocorticoid receptor target and participates in glucocorticoid-regulated triglyceride metabolism. J. Biol. Chem. 284, 25593-25601 (2009).
26. Liu, G. at al. Nephl and nephrin interaction in the slit diaphragm is an important determinant of glomerular permeability. J. Clin. Invest. 112, 209-221 (2003).
27. Dijkman, H.B.P.M., Mentzel, S., de Jong, A.S. & Assmann, K.J.M. RNA in situ hybridization using digoxigenin-labeled cRNA probes. Biochemica 2, 23-27 (1995).
28. Isogai, S., Mogami, K., Shiina, N. & Yoshino, G. Initial ultrastructural changes in pore size and anionic sites of the glomerular basement membrane in streptozotocin-induced diabetic rats and their prevention by insulin treatment. Nephron. 83, 53-58 (1999).
29. Bakker, W.W. et al. Altered activity of plasma hemopexin in patients with minimal change disease in relapse. Pediatr. Nephrol. 20, 1410-1415 (2005).
30. Graves, R.A., Tontonoz, P., Platt, K.A., Ross, S.R. & Spiegelman, B.M. Identification of a fat cell enhancer: analysis of requirements for adipose tissue-specific gene expression. J. Cell Biochem. 49, 219-224 (1992).
31. Yoshida, K., Ono, M., Koishi, R. & Furukawa, H. Characterization of the 5' regulatory region of the mouse angiopoietin-like protein 4. Vet. Res. Commun. 28, 299-305 (2004).
32. Zeng, L. et al. HMG CoA reductase inhibition modulates VEGF-induced endothelial cell hyperpermeability by preventing RhoA activation and myosin regulatory light chain phosphorylation. FASEB J. 19, 1845-1847 (2005).
33. Romeo, S. et al. Population-based resequencing of ANGPTL4 uncovers variations that reduce triglyceride and increase HDL. Nature Genetics, 39, 513-517 (2007).
34. Yin, Wu et al. Genetic variation in Angptl4 provides insight into protein processing and function, J.Biol.Chem., 284, 13213-13222 (2009).

## Claims

1. Angptl4 polypeptide for use in treatment or prevention of nephrotic syndrome.

2. An Angptl4 polypeptide for use in treatment or prevention of nephrotic syndrome containing the amino acid sequence of one of SEQ ID NOS: 9 or 10.

3. The Angptl4 polypeptide for use in treatment or prevention of nephrotic syndrome of claim 1, wherein the nephrotic syndrome is characterised as minimal change disease, focal segmental glomerulosclerosis, membranous nephropathy/membranous glomerulonephritis, membranoproliferative glomerulonephritis or a diabetic condition.

4. The Angptl4 polypeptide for use in treatment or prevention of nephrotic syndrome of claim 1, wherein said Angptl4 polypeptide comprises the sequence of SEQ ID NOs: 1 or 3.

5. The Angptl4 polypeptide for use according to claim 1, wherein said Angpt14 polypeptide is modified to have decreased lipoprotein lipase inhibitory activity as compared to wild-type Angpt14 polypeptide, is resistant to cleavage, or a combination of the foregoing.

6. The Angptl4 polypeptide for use in treatment or prevention of nephrotic syndrome of claim 2 or claim 5, wherein the Angptl4 polypeptide contains an amino acid substitution at position 40 with respect to the wild-type Angpt14 polypeptide.

7. The Angptl4 polypeptide for use in treatment or prevention of nephrotic syndrome of claim 2 or claim 5, wherein the Angpt14 polypeptide contains a E40K substitution, a E40A substitution, a D39K, a D39A substitution or a combination of the foregoing.

8. The Angptl4 polypeptide for use in treatment or prevention of nephrotic syndrome of claim 2 or claim 5, wherein the Angptl4 polypeptide contains a E40K substitution, a E40A substitution, a D39K substitution, a D39A substitution.

9. The Angpt14 polypeptide for use in treatment or prevention of nephrotic syndrome of claim 2 or claim 5, wherein the Angpt14 polypeptide contains at least one amino acid substitution, wherein the arginine residue at one or more of positions 161, 162 and 164 is substituted with D, R, K, G, A, V or S and the lysine residue at position 163 is substituted with D, R, K, G, A, V or S.

10. The Angptl4 polypeptide for use in treatment or prevention of nephrotic syndrome of claim 5, wherein the Angptl4 polypeptide contains an R161RKR164 to a G161 SGS164 substitution.

11. The Angptl4 polypeptide for use in treatment or prevention of nephrotic syndrome of claim 2 or claim 5, wherein the Angptl4 polypeptide has the sequence of SEQ ID NOS: 9 or 10, wherein X39 is D, X40 is A or K, X76 and X80 are C and X161 , X162, X163 and X164 are independently substituted with D, R, K, G, A, V or S, optionally provided that at least one of X161, X162, X163 and X164 is an amino acid not found in SEQ ID NOS: 1 or 3.

12. The Angptl4 polypeptide for use in treatment or prevention of nephrotic syndrome of claim 2 or claim 5, wherein the Angptl4 polypeptide has the sequence of SEQ ID NOS: 9 or 10, wherein X39 is D, X40 is A or K, one of X76 and X80 is A or S and the other of X76 and X80 is C and X161, X162, X 163 and X164 are independently substituted with D, R, K, G, A, V or S, optionally provided that at least one of X161 , X162, X163 and X164 is an amino acid not found in SEQ ID NOS: 1 or 3.

13. The Angpt14 polypeptide for use in treatment or prevention of nephrotic syndrome of claim 2 or claim 5, wherein the Angpt14 polypeptide has the sequence of SEQ ID NOS: 9 or 10, wherein X39 is A or K, X40 is E, X76 and X80 are C and X 161 , X162, X163 and X164 are independently substituted with D, R, K, G, A, V or S, optionally provided that at least one of X161, X162, X163 and X164 is an amino acid not found in SEQ ID NOS: 1 or 3.

14. The Angpt14 polypeptide for use in treatment or prevention of nephrotic syndrome of claim 2 or claim 5, wherein the Angpt14 polypeptide has the sequence of SEQ ID NOS: 9 or 10, wherein X39 is D, X40 is K, X76 and X80 are C and X161, X162, X163 and X164 are independently substituted with D, R, K, G, A, V or S, optionally provided that at least one of X161, X162, X163 and X164 is an amino acid not found in SEQ ID NOS: 1 or 3.

15. The Angpt14 polypeptide for use in treatment or prevention of nephrotic syndrome of claim 2 or claim 5, wherein the Angpt14 polypeptide has the sequence of SEQ ID NOS: 9 or 10, wherein X39 is D, X40 is K, one of X76 and X80 is A or S and the other of X76 and X80 is C and X161, X162, X163 and X164 are independently substituted with D, R, K, G, A, V or S, optionally provided that at least one of X161, X162, X163 and X164 is an amino acid not found in SEQ ID NOS: 1 or 3.

16. The Angptl4 polypeptide for use in treatment or prevention of nephrotic syndrome of claim 1 or claim 2, wherein the Angptl4 polypeptide is sialylated.

17. Use of an Angpt14 polypeptide as defined in any of claims 1 to 15 in the manufacture of a medicament for nephrotic syndrome.

## Patentansprüche

1. Angptl4-Polypeptid zur Anwendung bei der Behandlung oder Verhütung des nephrotischen Syndroms.

2. Angptl4-Polypeptid zur Anwendung bei der Behandlung oder Verhütung des nephrotischen Syndroms, welches die Aminosäurensequenz von einem von SEQ ID NOS: 9 oder 10 enthält.

3. Angptl4-Polypeptid zur Anwendung bei der Behandlung oder Verhütung des nephrotischen Syndroms nach Anspruch 1, wobei das nephrotische Syndrom gekennzeichnet ist als Minimal-Change-Erkrankung, fokal segmentale Glomerulosklerose, membranöse Nephropathie/membranöse Glomerulonephritis, membranoproliferative Glomerulonephritis oder eine diabetische Erkrankung.

4. Angptl4-Polypeptid zur Anwendung bei der Behandlung oder Verhütung des nephrotischen Syndroms nach Anspruch 1, wobei das Angptl4-Polypeptid die Sequenz von SEQ ID NOs: 1 oder 3 umfasst.

5. Angptl4-Polypeptid zur Anwendung nach Anspruch 1, wobei das Angptl4-Polypeptid modifiziert ist, sodass es im Vergleich zu Wildtyp-Angptl4-Polypeptid verminderte Lipoprotein-Lipase-hemmende Aktivität aufweist, resistent gegen Spaltung ist, oder eine Kombination des Vorangehenden.

6. Angptl4-Polypeptid zur Anwendung bei der Behandlung oder Verhütung des nephrotischen Syndroms nach Anspruch 2 oder Anspruch 5, wobei das Angptl4-Polypeptid in Bezug auf das Wildtyp-Angptl4-Polypeptid eine Aminosäuren-Substitution in Position 40 enthält.

7. Angptl4-Polypeptid zur Anwendung bei der Behandlung oder Verhütung des nephrotischen Syndroms nach Anspruch 2 oder Anspruch 5, wobei das Angptl4-Polypeptid eine E40K-Substitution, eine E40A-Substitution, eine D39K-, eine D39A-Substitution oder eine Kombination der Vorgenannten enthält.

8. Angptl4-Polypeptid zur Anwendung bei der Behandlung oder Verhütung des nephrotischen Syndroms nach Anspruch 2 oder Anspruch 5, wobei das Angptl4-Polypeptid eine E40K-Substitution, eine E40A-Substitution, eine D39K-Substitution, eine D39A-Substitution enthält.

9. Angptl4-Polypeptid zur Anwendung bei der Behandlung oder Verhütung des nephrotischen Syndroms nach Anspruch 2 oder Anspruch 5, wobei das Angptl4-Polypeptid mindestens eine Aminosäuren-Substitution enthält, wobei der Argininrückstand in einer oder mehr der Positionen 161, 162 und 164 mit D, R, K, G, A, V oder S substituiert ist und der Lysinrückstand in Position 163 mit D, R, K, G, A, V oder S substituiert ist.

10. Angptl4-Polypeptid zur Anwendung bei der Behandlung oder Verhütung des nephrotischen Syndroms nach Anspruch 5, wobei das Angptl4-Polypeptid eine R161RKR164 bis eine G161SGS164-Substitution enthält.

11. Angptl4-Polypeptid zur Anwendung bei der Behandlung oder Verhütung des nephrotischen Syndroms nach Anspruch 2 oder Anspruch 5, wobei das Angptl4-Polypeptid die Sequenz von SEQ ID NOS: 9 oder 10 aufweist, wobei X39 D ist, X40 A oder K ist, X76 und X80 C sind, und X161, X162, X163 und X164 unabhängig durch D, R, K, G, A, V oder S substituiert sind, optional mit der Maßgabe, dass mindestens eines von X161, X162, X163 und X164 eine Aminosäure ist, die sich nicht in SEQ ID NOS:1 oder 3 findet.

12. Angptl4-Polypeptid zur Anwendung bei der Behandlung oder Verhütung des nephrotischen Syndroms nach Anspruch 2 oder Anspruch 5, wobei das Angptl4-Polypeptid die Sequenz von SEQ ID NOS: 9 oder 10 aufweist, wobei X39 D ist, X40 A oder K ist, eines von X76 und X80 A oder S ist und das andere von X76 und X80 C ist, und X161, X162, X163 und X164 unabhängig mit D, R, K, G, A, V oder S substituiert sind, optional mit der Maßgabe, dass mindestens eines von X161, X162, X163 und X164 eine Aminosäure ist, die sich nicht in SEQ ID NOS:1 oder 3 findet.

13. Angptl4-Polypeptid zur Anwendung bei der Behandlung oder Verhütung des nephrotischen Syndroms nach Anspruch 2 oder Anspruch 5, wobei das Angptl4-Polypeptid die Sequenz von SEQ ID NOS: 9 oder 10 aufweist, wobei X39 A oder K ist, X40 E ist, X76 und X80 C sind und X161, X162, X163 und X164 unabhängig mit D, R, K, G, A, V oder S substituiert sind, optional mit der Maßgabe, dass mindestens eines von X161, X162, X163 und X164 eine Aminosäure ist, die sich nicht in SEQ ID NOS:1 oder 3 findet.

14. Angptl4-Polypeptid zur Anwendung bei der Behandlung oder Verhütung des nephrotischen Syndroms nach Anspruch 2 oder Anspruch 5, wobei das Angptl4-Polypeptid die Sequenz von SEQ ID NOS: 9 oder 10 aufweist, wobei X39 D ist, X40 K ist, X76 und X80 C sind und X161, X162, X163 und X164 unabhängig mit D, R, K, G, A, V oder S substituiert sind, optional mit der Maßgabe, dass mindestens eines von X161, X162, X163 und X164 eine Aminosäure ist, die sich nicht in SEQ ID NOS:1 oder 3 findet.

15. Angptl4-Polypeptid zur Anwendung bei der Behandlung oder Verhütung des nephrotischen Syndroms nach Anspruch 2 oder Anspruch 5, wobei das Angptl4-Polypeptid die Sequenz von SEQ ID NOS: 9 oder 10 aufweist, wobei X39 D ist, X40 K ist, eines von X76 und X80 A oder S ist und das andere von X76 und X80 C ist, und X161, X162, X163 und X164 unabhängig mit D, R, K, G, A, V oder S substituiert sind, optional mit der Maßgabe, dass mindestens eines von X161, X162, X163 und X164 eine Aminosäure ist, die sich nicht in SEQ ID NOS:1 oder 3 findet.

16. Angptl4-Polypeptid zur Anwendung bei der Behandlung oder Verhütung des nephrotischen Syndroms nach Anspruch 1 oder Anspruch 2, wobei das Angptl4-Polypeptid sialysiert ist.

17. Anwendung eines Angptl4-Polypeptids, wie in einem der Ansprüche 1 bis 15 definiert, bei der Herstellung eines Medikaments für das nephrotische Syndrom.

## Revendications

1. Polypeptide Angptl4 pour une utilisation dans le traitement ou la prévention du syndrome néphrotique.

2. Polypeptide Angptl4 pour une utilisation dans le traitement ou la prévention du syndrome néphrotique contenant la séquence d'acides aminés de l'une des SEQ ID NO : 9 ou 10.

3. Polypeptide Angptl4 pour une utilisation dans le traitement ou la prévention du syndrome néphrotique selon la revendication 1, dans laquelle le syndrome néphrotique est caractérisé comme une maladie à changement minime, une glomérulosclérose segmentaire focale, une néphropathie membranaire/une glomérulonéphrite membranaire, une glomérulonéphrite membranoproliférative ou un état diabétique.

4. Polypeptide Angptl4 pour une utilisation dans le traitement ou la prévention du syndrome néphrotique selon la revendication 1, dans laquelle ledit polypeptide Angptl4 comprend la séquence de SEQ ID NO : 1 ou 3.

5. Polypeptide Angptl4 pour une utilisation selon la revendication 1, dans laquelle ledit polypeptide Angptl4 est modifié pour avoir une activité inhibitrice de la lipoprotéine lipase diminuée par rapport au polypeptide Angptl4 de type sauvage, est résistant au clivage, ou une combinaison de ce qui précède.

6. Polypeptide Angptl4 pour une utilisation dans le traitement ou la prévention du syndrome néphrotique selon la revendication 2 ou la revendication 5, dans laquelle le polypeptide Angptl4 contient une substitution d'acide aminé à la position 40 par rapport au polypeptide Angptl4 de type sauvage.

7. Polypeptide Angptl4 pour une utilisation dans le traitement ou la prévention du syndrome néphrotique selon la revendication 2 ou la revendication 5, dans laquelle le polypeptide Angptl4 contient une substitution E40K, une substitution E40A, une D39K, une substitution D39A ou une combinaison de ce qui précède.

8. Polypeptide Angptl4 pour une utilisation dans le traitement ou la prévention du syndrome néphrotique selon la revendication 2 ou la revendication 5, dans laquelle le polypeptide contient une substitution E40K, une substitution E40A, une substitution D39K, une substitution D39A.

9. Polypeptide Angptl4 pour une utilisation dans le traitement ou la prévention du syndrome néphrotique selon la revendication 2 ou la revendication 5, dans laquelle le polypeptide Angptl4 contient au moins une substitution d'acide aminé, dans lequel le résidu d'arginine à une ou plusieurs des positions 161, 162 et 164 est substitué par D, R, K, G, A, V ou S et le résidu de lysine à la position 163 est substitué par D, R, K, G, A, V ou S.

10. Polypeptide Angplt4 pour une utilisation dans le traitement ou la prévention du syndrome néphrotique selon la revendication 5, dans laquelle le polypeptide Angptl4 contient une substitution de R161RKR164 par G161 SGS164.

11. Polypeptide Angptl4 pour une utilisation dans le traitement ou la prévention du syndrome néphrotique selon la revendication 2 ou la revendication 5, dans laquelle le polypeptide Angplt4 a la séquence de SEQ ID NO : 9 ou 10, dans laquelle X39 est D, X40 est A ou K, X76 et X80 sont C et X161, X162, X163 et X164 sont indépendamment substitués par D, R, K, G, A, V ou S, éventuellement sous réserve que l'un au moins de X161, X162, X163 et X164 est un acide aminé non trouvé dans SEQ ID NO : 1 ou 3.

12. Polypeptide Angptl4 pour une utilisation dans le traitement ou la prévention du syndrome néphrotique selon la revendication 2 ou la revendication 5, dans laquelle le polypeptide Angptl4 a la séquence de SEQ ID NO : 9 ou 10, dans laquelle X39 est D, X40 est A ou K, l'un de X76 et X80 est A ou S et l'autre de X76 et X80 est C et X161, X162, X163 et X164 sont indépendamment substitués par D, R, K, G, A, V ou S, éventuellement sous réserve que l'un au moins de X161, X162, X163 et X164 est un acide aminé non trouvé dans SEQ ID NO : 1 ou 3.

13. Polypeptide Angptl4 pour une utilisation dans le traitement ou la prévention du syndrome néphrotique selon la revendication 2 ou la revendication 5, dans lequel le polypeptide Angptl4 a la séquence de SEQ ID NO : 9 ou 10, dans lequel X39 est A ou K, X40 est E, X76 et X80 sont C et X161, X162, X163 et X164 sont indépendamment substitués par D, R, K, G, A, V ou S, éventuellement sous réserve que l'un au moins de X161, X162, X163 et X164 est un acide aminé non trouvé dans SEQ ID NO : 1 ou 3.

14. Polypeptide Angptl4 pour une utilisation dans le traitement ou la prévention du syndrome néphrotique selon la revendication 2 ou la revendication 5, dans lequel le polypeptide Angptl4 a la séquence de SEQ ID NO : 9 ou 10, dans lequel X39 est D, X40 est K, X76 et X80 sont C et X161, X162, X163 et X164 sont indépendamment substitués par D, R, K, G, A, V ou S, éventuellement sous réserve que l'un au moins de X161, X162, X163 et X164 est un acide aminé non trouvé dans SEQ ID NO : 1 ou 3.

15. Polypeptide Angptl4 pour une utilisation dans le traitement ou la prévention du syndrome néphrotique selon la revendication 2 ou la revendication 5, dans laquelle le polypeptide Angptl4 a la séquence de SEQ ID NO : 9 ou 10, dans lequel X39 est D, X40 est K, l'un de X76 et X80 est A ou S et l'autre de X76 et X80 est C et X161, X162, X163 et X164 sont indépendamment substitués par D, R, K, G, A, V ou S, éventuellement sous réserve que l'un au moins de X161, X162, X163 et X164 est un acide aminé non trouvé dans SEQ ID NO : 1 ou 3.

16. Polypeptide Angptl4 pour une utilisation dans le traitement ou la prévention du syndrome néphrotique selon la revendication 1 ou la revendication 2, dans lequel le polypeptide Angptl4 est sialylé.

17. Utilisation d'un polypeptide Angptl4 tel que défini dans l'une quelconque des revendications 1 à 15 dans la préparation d'un médicament pour le syndrome néphrotique.
